# EUROPEAN PATENT SPECIFICATION

(11) **EP 0 276 485 B1**
(45) Date of publication and mention of the grant of the patent: **12.06.2002**
(21) Application number: 87119344.7
(22) Date of filing: 30.12.1987
(51) Int. Cl.: C07H 15/203, C12P 19/44, A61K 31/70, C12N 1/20, C12P 19/00

(54) **Dihydro derivatives of LL-E33288 antibiotics**
Dihydroderivate der Antibiotika vom Typ LL-E33288
Dérivés dihydro de composés antibiotiques du type LL-E33288

(30) Priority: 30.01.1987 US 4154; 30.01.1987 US 4153; 30.01.1987 US 9321
(43) Date of publication of application: 03.08.1988
(60) Divisional application: 01123067.9
(73) Proprietor: AMERICAN CYANAMID COMPANY, Wayne, NJ 07470-8426 (US)
(72) Inventor: Lee, May Dean-Ming, Monsey New York 10952 (US); Greenstein, Michael, Suffern New York 10901 (US); Labeda, David Paul, Peoria Illinois 61614 (US); Fantini, Amedeo Alexander, New City New York 10956 (US)
(74) Representative: Wächtershäuser, Günter, Prof. Dr.

(56) References cited:
- EP-A- 0 182 152
- GB-A- 2 179 649
- J. AM. CHEM. SOC., vol. 109, 1987, pages 3466-3468, American Chemical Society, US; M.D. LEE et al.: "Calichemicins, a novel family of antitumor antibiotics. 2. Chemistry and structure of calichemicin gamma(1)".

## Description

This invention relates to new antibacterial and antitumor agents designated pseudoaglycone and dihydro derivatives of LL-E33288α₁-I, LL-E33288α₂-I, LL-E33288α₃-I, LL-E33288β₁-I, LL-E33288β₂-I, LL-E33288γ₁-I, and LL-E33288δ₁-I, LL-E33288α₁-Br, LL-E33288α₂-Br, LL-E33288α₃-Br, LL-E33288β₁-Br, LL-E33288β₂-Br LL-E33288γ₁-Br and LL-E33288δ₁-Br to their production by fermentation, to methods for their recovery and concentration from crude solutions and to processes for their purification. The present invention includes within its scope the anti-bacterial and antitumor agents in dilute form, as crude concentrates, as a complex of various or all components, in pure form as individual components and novel strains of Micromonospora.

The LL-E33288 antibiotics are closely related compounds. The fourteen antibiotics are recovered from fermentation and are initially obtained as a mixture, hereinafter either the LL-E33288 complex, the LL-E33288 Iodo-complex or the LL-E33288 Bromo-complex. In general, the iodine containing components of the LL-E33288 antibiotics (e.g., α₁-I, α₂-I, α₃-I, β₁-I, β₂-I, γ₁-I, and δ₁-I) are found only in fermentation using media containing inorganic or organic iodide while the bromine containing components (e.g., α₁-Br, α₂-Br, α₃-Br, α₄-Br, β₁-Br, β₂-Br, and γ₁-Br) are found only in fermentations using media containing inorganic or organic bromide. While the ratio of components in the LL-E33288 complex will vary, depending upon the fermentation of both the bromine and the iodine containing antibiotics, LL-E33288β₁, LL-E33288γ₁ and LL-E33288δ₁-I, are generally the major components, together accounting for approximately 90% of the complex. LL-E33288α₁, LL-E33288α₂, LL-E33288α₃, LL-E33288α₄ and LL-E33288β₂ are minor components, together accounting for approximately 10% of the complex.

The LL-E33288 antibiotics are active against gram-positive and gram-negative bacteria. Each of the components were also found to be active in a modification of the Biochemical Induction Assay [Elespuru, R. and Yarmolinsky, M., Environmental Mutagenesis, 1, 65-78 (1978)], a test which specifically measures the ability of an agent to directly or indirectly initiate DNA damage. In this assay, both LL-E33288γ₁-I and LL-E33288δ₁-I were active at concentrations lower than 1x10⁻⁶ mcg/ml.

### DETAILED DESCRIPTION OF THE INVENTION

Proposed structures of some of the LL-E33288 anti-biotics are disclosed below.

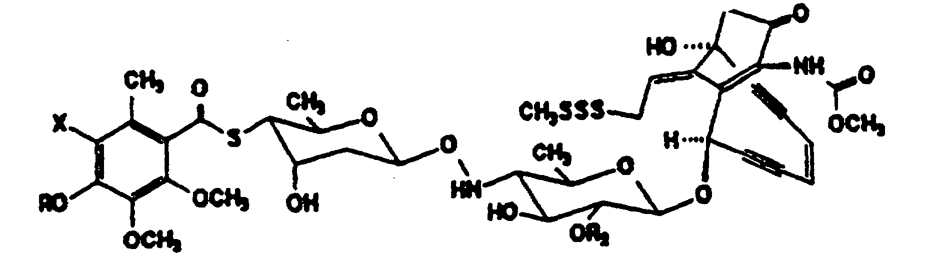

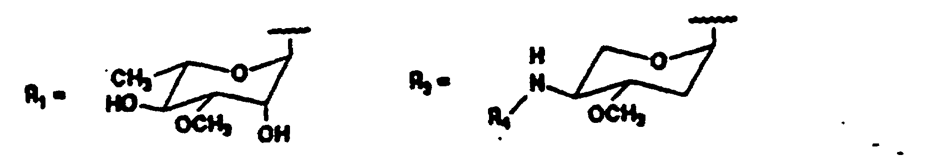

| E33288 | X | R | R₂ | R₄ |
|---|---|---|---|---|
| α₂^{I} | I | H | R₃ | C₂H₅ |
| α₃^{I} | I | R₁ | H | |
| β₁^{I} | I | R₁ | R₃ | (CH₃)₂CH |
| γ₁^{I} | I | R₁ | R₃ | C₂H₅ |
| δ₁^{I} | I | R₁ | R₃ | CH₃ |
| β₁^{Br} | Br | R₁ | R₃ | (CH₃)₂CH |
| γ₁^{Br} | Br | R₁ | R₃ | C₂H₅ |
| α₂^{Br} | Br | H | R₃ | C₂H₅ |
| α₃^{Br} | Br | R₁ | H | |

The dihydro-LL-E33288-pseudoaglycone has the following proposed structure: 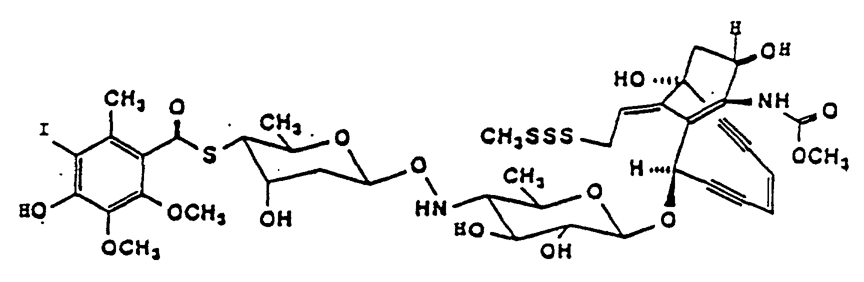 and the following physico-chemical characteristics:
a) molecular weight: 1052, determined by FAB-MS;
b) ultraviolet absorption spectrum: as shown in Figure I (methanol);
c) infrared absorption spectrum: as shown in Figure II (KBr disc);
d) proton magnetic resonance spectrum: as shown in Figure III (300MHz, CDCl₃); and
e) carbon-13 magnetic resonance spectrum: as shown in Figure IV (75MHz, CDCl₃).

When a dilute methanolic solution of an iodinated LL-E33288 component such as LL-E33288γ₁-I is treated with a cation exchange resin such as Dowex(R) 50W-X8(H form) the biologically active pseudoaglycone, having the following physico-chemical characteristics and proposed structure is obtained.
a) Molecular weight: 1050, determined by FAB-MS;
b) Molecular formula: C₄₀H₄₇N₂O₁₅IS₄, exact mass for M+Na was determined by high resolution FAB-MS t be 1073.0810 for C₄₀H₄₇N₂O₁₅IS₄Na;
c) Ultraviolet absorption spectra: as shown in Figure V (methanol; 0.1N HCl; 0.1N NaOH);
d) Infrared absorption spectrum: as shown in Figure VI (KBr disc);
e) Proton magnetic resonance spectrum: as shown in Figure VII (300MHz, CDCl₃); and
f) Carbon-13 magnetic resonance spectrum: as shown in Figure VIII (75.43MHz, CDCl₃, ppm from TMS) significant peaks as listed in Table I.

**TABLE I**

| Peak No. | PPM | Peak No. | PPM |
|---|---|---|---|
| 1 | 17.8 q | 21 | 84.4 s |
| 2 | 19.1 q | 22 | 87.5 s |
| 3 | 22.8 q | 23 | 98.7 s |
| 4 | 24.7 q | 24 | 99.7 d |
| 5 | 36.8 t | 25 | 100.4 s |
| 6 | 39.1 t | 26 | 103.5 d |
| 7 | 51.6 d | 27 | 124.1 d |
| 8 | 53.3 t | 28 | 124.2 d |
| 9 | 53.6 q | 29 | 126.8 s |
| 10 | 61.0 q | 30 | 127.5 d |
| 11 | 61.5 q | 31 | 130.6 s |
| 12 | 67.2 d | 32 | 133.2 s |
| 13 | 68.2 d | 33 | 136.3 s |
| 14 | 69.1 d | 34 | 136.4 s |
| 15 | 69.6 d | 35 | 140.7 s |
| 16 | 70.1 d | 36 | 148.8 s |
| 17 | 71.3 d | 37 | 150.9 s |
| 18 | 72.5 s | 38 | 154.3 s |
| 19 | 74.5 d | 39 | 191.8 s |
| 20 | 83.9 s | 40 | 192.0 s |

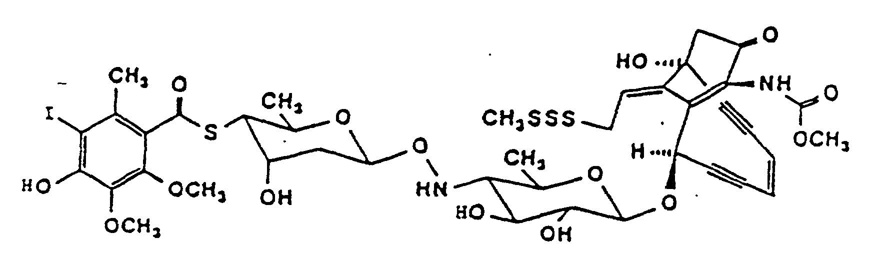

It has now been discovered that all of the aforementioned LL-E33288 components produced by NRRL-15839 and 15975 are also produced by a newly derived mutant named LL-E33288-UV 784, in much higher yields.

### Derivation of Mutant LL-E33288-UV 784

Vegetative growth from isolate UV 610(3) (see following diagram) was prepared as employed for fermentation and used to inoculate a flask of medium consisting of peptone, dextrose, molasses and water. The medium was supplemented with LL-E33288β₁-Br at a concentration of about 8 µg/ml. A number of platings were done from this flask and a resistant population was obtained on the seventh day. A total of 97 colonies (R1 to R97) were isolated. Isolate R66 became NRRL-15975. Isolate R80 is essentially similar to R66 in its biosynthetic potential.

Isolate R80 was then used as the starting culture from which a spore suspension was prepared and exposed to relatively high concentrations of the LL-E33288 complex, the purpose being to obtain isolates resistant to the LL-E33288 antibiotics and thereby improve production yields.

One survivor, labeled T₂ did produce greater yields of LL-E33288β₁-Br and LL-E33288γ₁-I in flask fermentations.

A spore suspension of T₂ was prepared and exposed to UV-irradiation. A total of 131 colonies were then isolated (UV 703 to UV 834), fermented and assayed. From this group, isolate UV 784 was selected for its activity in flask fermentations. Isolate UV 784, when fermented in the iodine containing medium, produced approximately double the yield of R66 (NRRL-15975).

Diagram of UV 784 Derivation
E-33288 (Wild type) (NRRL-15839)
Natural selection
NS6
Ultraviolet radiation
UV610
Natural selection
UV610(3)
Exposure to LL-E33288β1-Br
R80
R66 (NRRL-15975)
Exposure to LL-E33288 complex
T2
Ultraviolet radiation
UV 784

The mutant LL-E33288 UV 784 is maintained by that number in the culture collection of the Medical Research Division, American Cyanamid Company, Pearl River, New York. A viable culture of this microorganism has been deposited with the Culture Collection Laboratory, Northern Regional Research Canter, U. S. Department of Agriculture, Peoria, Illinois on December 3, 1986, and has been added to its permanent collection. Access to said culture, under strain designation NRRL-18149, during pendency of the instant application shall be available to one determined by the Commissioner of Patents and Trademarks to be entitled thereto under C.F.R. §1.14 and 35 U.S.C. §122, and all restrictions on availability to the public of such culture will be irrevocably removed upon grant of a U. S. Patent on the instant application.

It is to be understood that for the production of these new antibacterial and anti-tumor agents the present invention is not limited to this particular organisms or to organisms fully answering the above growth microscopic characteristics which were given for illustrative purposes only. In fact, it is desired and intended to include the use of mutants produced from these organisms by various means such as exposure to X-radiation, ultraviolet radiation, N'-methyl-N- nitro-N-nitrosoquanidine, actinophages and the like.

The in vitro antibacterial activity of dihydro LL-E33288gamma l-I was determined against a spectrum of gram-positive and gram-negative bacteria by a standard agar dilution method. Mueller-Hinton agar containing twofold decreasing concentrations of the antibiotics was poured into petri plates. The agar surfaces were inoculated with 1 to 5 x 10⁴ colony forming units of bacteria by means of a Steers replicating device. The lowest concentration of LL-E33288 component that inhibited growth of a bacterial strain after about 18 hours of incubation at approximately 35°C was recorded as the minimal inhibitory concentration (MIC) for that strain. The results are summarized in Table II.

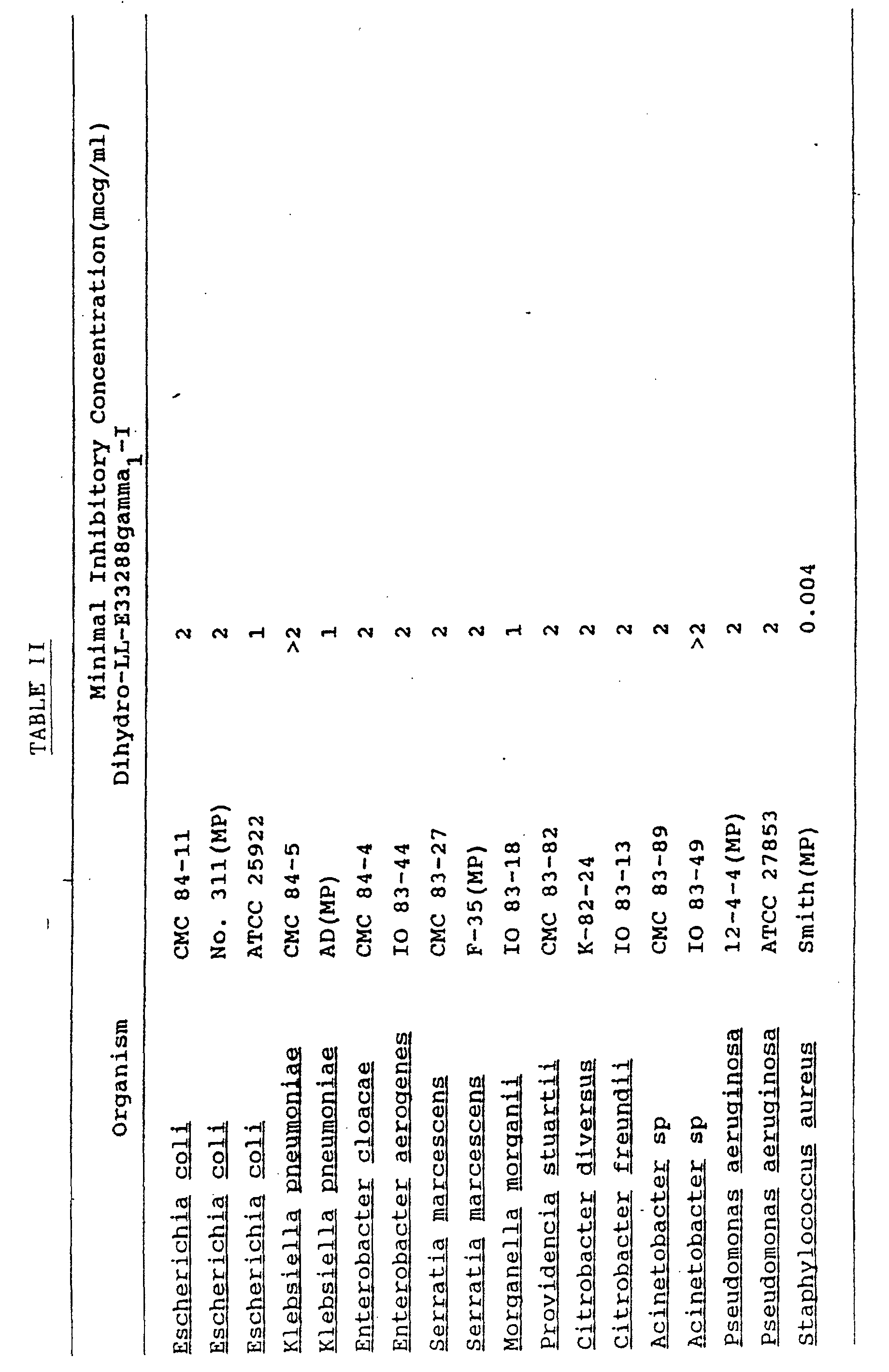

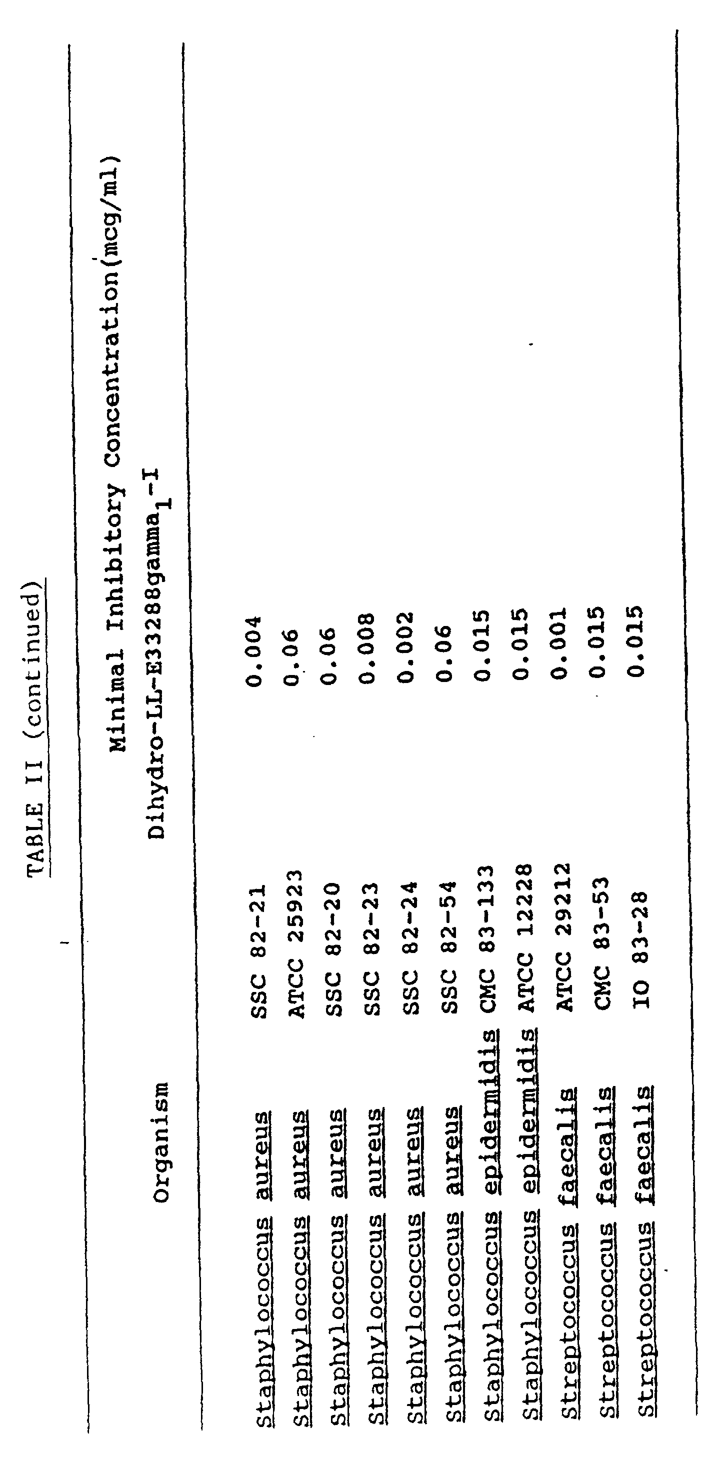

According to the present invention there are provided :

The dihydro derivative of the iodinated pseudoaglycone of LL-E33288 of the formula:

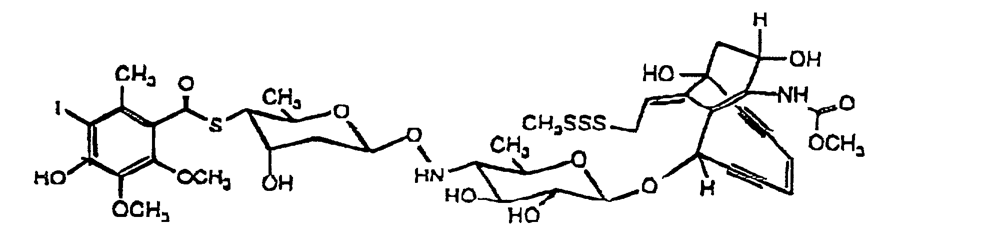

The dihydro derivative of the brominated pseudoaglycone of LL-E33288 of the formula:

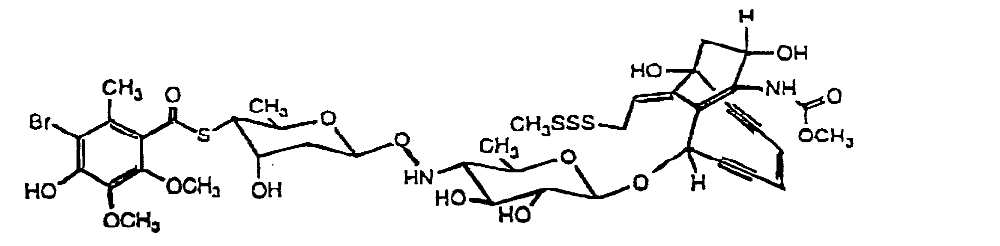

The dihydro derivative of LL-E33288α₂-Br of the formula:

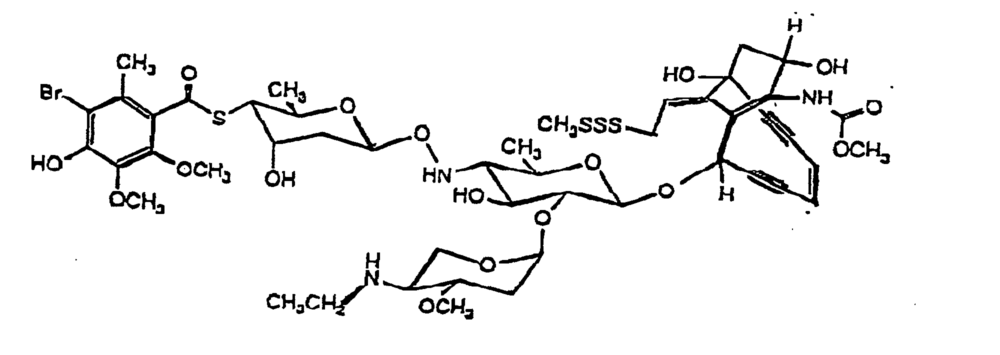

The dihydro derivative of LL-E33288α₃-Br of the formula;

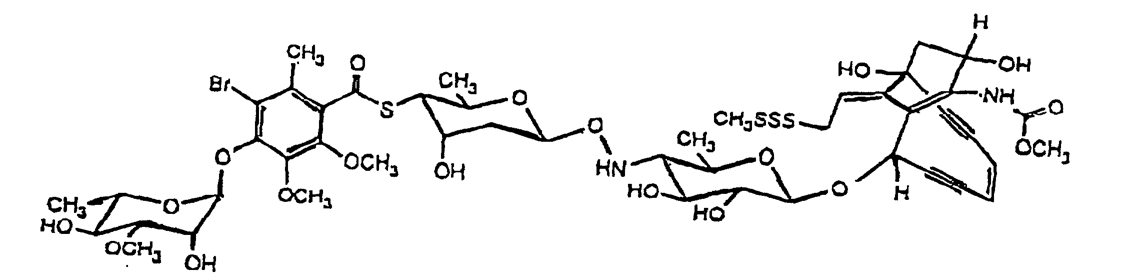

The dihydro derivative of LL-E33288β₁-Br of the formula:

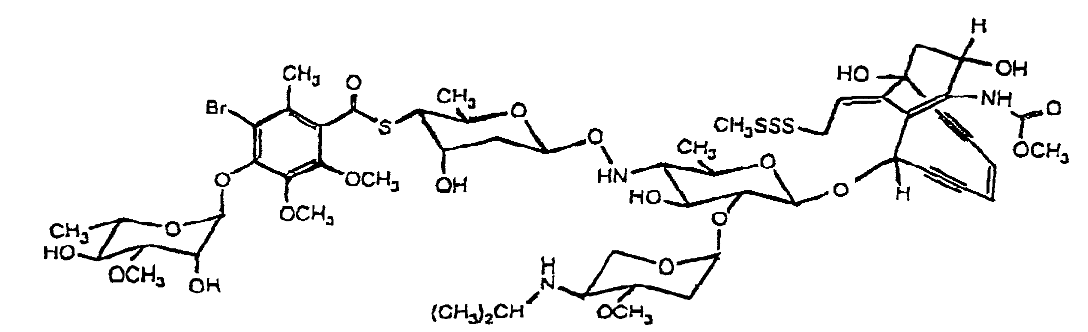

The dihydro derivative of LL-E33288γ₁-Br of the formula:

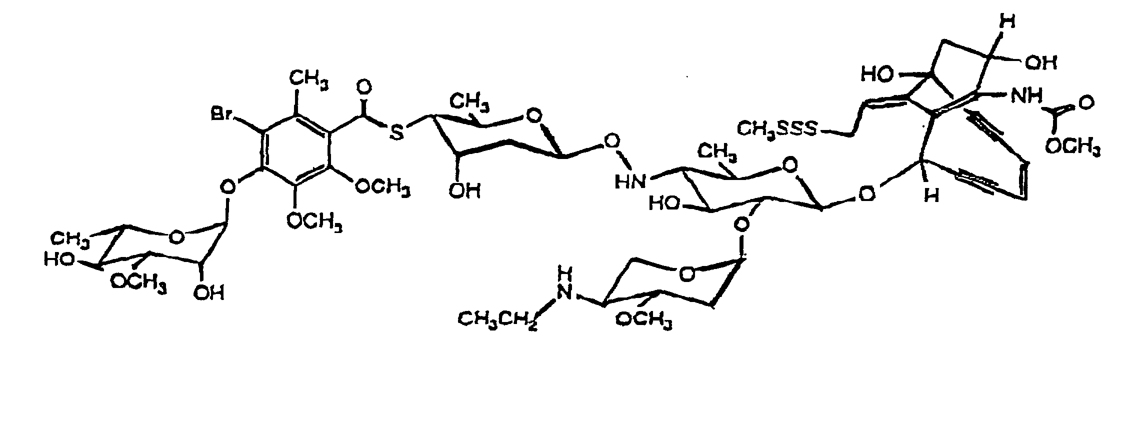

The dihydro derivative of LL-E33288α₂-I of the formula:

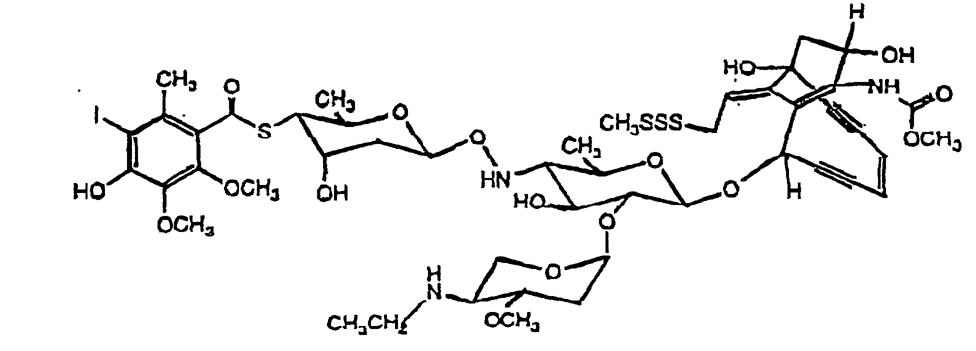

The dihydro derivative of LL-E33288α₃-I of the formula:

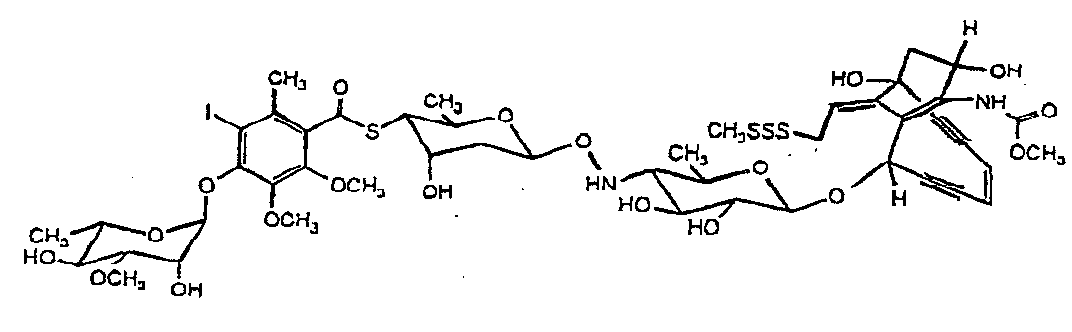

The dihydro derivative of LL-E33288β₁-I of the formula:

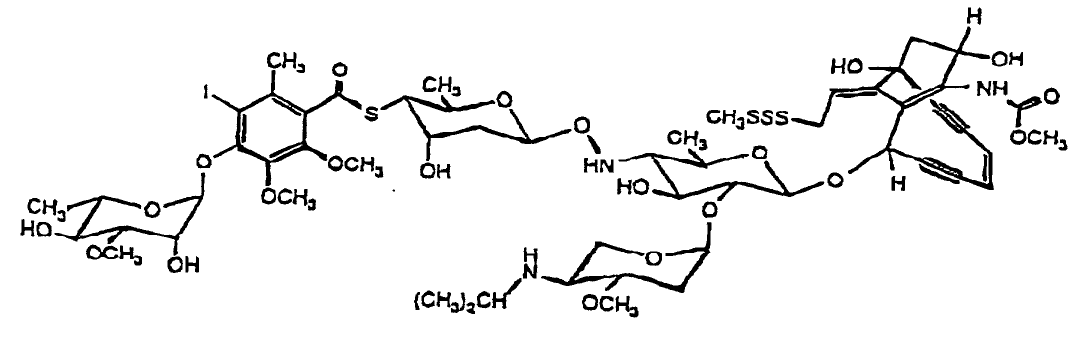

The dihydro derivative of LL-E33288γ₁-I of the formula:

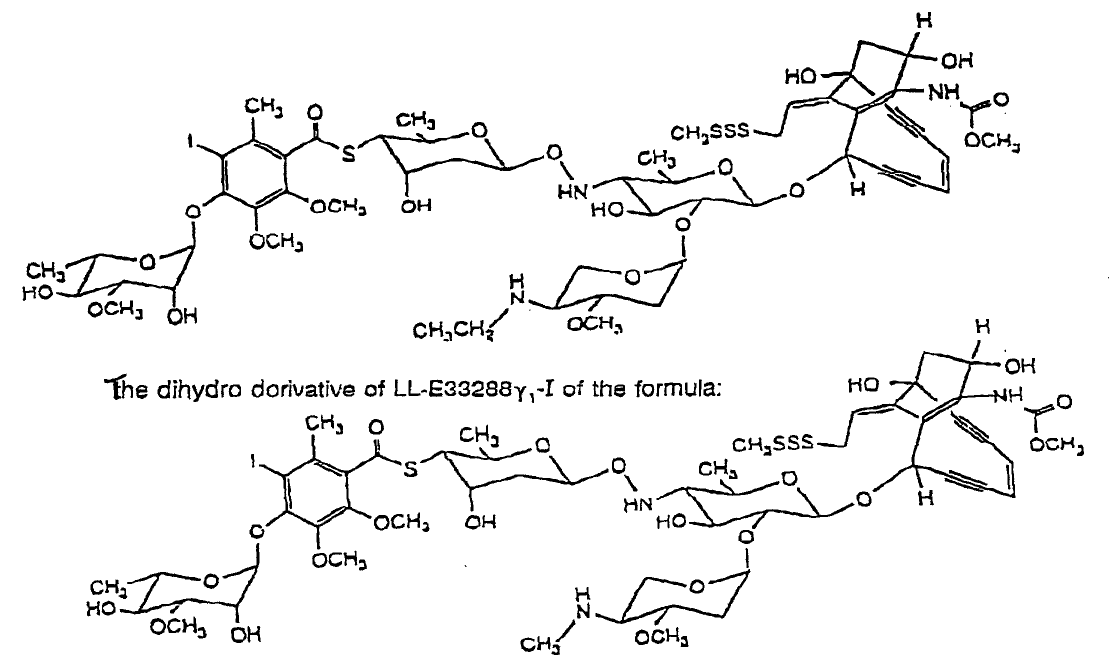

An LL-E33288 antitumor antibiotic obtainable inter alia by:
aerobically fermenting a liquid medium containing assimilable sources of carbon, nitrogen, iodine and inorganic salts, which medium has been inoculated with a viable culture of the organism **Micromonosporal echinospora** ssp, **calichensis** mutant NRRL-18149 or NRRL-15839 or mutants of NRRL-18149, until substantial antibiotic activity is imparted;
maintaining said fermentation culture at a temperature of about 24-32°C for a period of approximately 90-200 hours; harvesting the mash and recovering the antibiotic; adding an alkyl iodide to an ethanolic solution of the antibiotic; cooling the solution to ice-bath temperature;
adding an ethanolic solution of sodiuxm borohydride; decomposing the borate complex by the addition of acetic acid, precipitating from an ethyl acetate solution by the addition of hexane and purifying by chromatography.

An antitumor antibiotic designated dihydro-LL-E33288-pseudoaglycone having the following physico-chemical characteristics:
a) molecular weight 1052 (determined by FAB-MS);
b) ultraviolet absorption spectrum as shown in Figure I (methanol);
c) infrared absorption spectrum as shown in Figure II (KBr disc);
d) proton magnetic resonance spectrum as shown in Figure III (300 MHz, CDCl₃); and
e) carbon-13 magnetic resonance spectrum as shown in Figure IV (75 MHz, CDCl₃).

A compound as defined above for use in treating bacterial infections in warm-blooded animals.

A compound as defined above for use in inhibiting the growth of tumors in a mammal.

A compound as defined above for use in regressing leukemia in a mammal.

The use of a compound as defined above in the manufacture of a medicament for treating bacterial infections in warm-blooded animals.

The use of a compound as defined above in the manufacture of a medicament for inhibiting the growth of tumors in a mammal.

A process for producing the dihydro derivative of the iodinated pseudoaglycone of LL-E33288, dihydro derivative of the brominated pseudoaglycone of LL-E33288, dihydro-LL-E33288 α₂-Br, dihydro-LL-E33288 α₃-Br, dihydro-LL-E33288 β₁-Br, dihydro-LL-E33288 γ₁-Br, dihydro-LL-E332B8 α₂-I, dihydro-LL-E33288 α₃-I, dihydro-LL-E33288 β₁-I, dihydro-LL-E33288 γ₁-I or dihydro-LL-E33288 δ₁-I as defined in any one of claims 1 to 5 which comprises adding an alkyl iodide to an ethanolic solution of the antibiotic, cooling the solution to ice-bath temperature, adding an ethanolic solution of sodium borohydride, decomposing the borate complex by the addition of acetic acid, precipitating from an ethyl acetate solution by the addition of hexane and purifying by chromatography.

A composition of matter in unit dosage form comprising an antibacterially effective amount of a compound as defined above.

### General Fermentation Conditions

Cultivation of Micromonospora echinospora NRRL-15839 or NRRL-15975 may be carried out in a wide variety of liquid culture media. Media which are useful for the production of these novel antibacterial and anti-tumor agents include an assimilable source of carbon, such as starch, sugar, molasses, glycerol, etc.; an assimilable source of nitrogen such as protein, protein hydrolysate, polypeptides, amino acids, corn steep liquor, etc.; and inorganic anions and cations, such as potassium, sodium, ammonium, calcium, sulfate, carbonate, phosphate, chloride, etc. and sources of either bromine (sodium bromide) or iodine (potassium iodide). Trace elements such as boron, molybdenum, copper, etc., are supplied as impurities of other-constituents of the media. Aeration in tanks and bottles is supplied by forcing sterile air through or onto the surface of the fermenting medium. Further agitation in tanks is provided by a mechanical impeller. An antifoam agent such as silicone may be added as needed.

### General Procedure for the Isolation and Separation of the Antibiotics - LL-E33288

The LL-E33288 antibiotics are recovered from the fermentation broth by extracting the whole mash with an organic solvent such as ethyl acetate or dichloromethane. The antibiotic complex, contained in the organic extract, is further purified by selective precipitation from lower hydrocarbons. The crude LL-E33288 antibiotic complex thus obtained is further purified and separated into the individual components by a series of column chromatographies using silica gel, Sephadex® LH-20 (Pharmacia Fine Chemicals) and c₁₈ bonded silica.

The invention will be described in greater detail in conjunction with the following non-limiting specific examples.

### Example 1

### Small Scale Preparation of LL-E33288 Complex from LL-E33288-UV 784, NRRL-18149

A suspension containing spores and mycelia was prepared from a slant of Micromonospora echinospora ssp. calichensis, LL-E33288-UV 784 (NRRL-18149) by adding 5-8 ml of water and scraping the surface of the slant. This suspension was used to inoculate 50 ml of a sterile medium of the following composition:

| | |
|---|---|
| Yeast extract | 0.5% |
| Beef extract | 0.3% |
| Tryptose | 0.5% |
| Dextrin | 2.4% |
| Dextrose | 0.5% |
| Calcium carbonate | 0.4% |
| Water qs | 100% |

The above medium, in a 250 ml Erlenmeyer flask, was incubated at 28°C on a rotary shaker at 200 rpm for 3-4 days thus providing stage I inoculum.

Stage I inoculum was used to inoculate 50 ml of the same sterile medium in a 250 ml baffled flask and incubated at 28°C on a rotary shaker at 250 rpm for 2 days, thus providing stage II inoculum.

Stage II inoculum was used to inoculate 100 ml of sterile fermentation medium of the following composition:

| | |
|---|---|
| Sucrose | 2.0% |
| Ferrous sulfate heptahydrate | 0.01% |
| Magnesium sulfate heptahydrate | 0.02% |
| Calcium carbonate | 0.25% |
| Peptone | 0.4% |
| Molasses | 0.25% |
| Potassium iodide* | 0.01% |
| Water qs | 100% |

| | |
|---|---|
| *Potassium bromide may be substituted to produce the bromo analogs. | |

The above medium in 500 ml baffled flasks was incubated at 28-30°C on a rotary shaker at 250 rpm for 6 days at which time the fermentation was harvested.

### Example 2

### Large Scale Fermentation of LL-E33288 Complex Using LL-E33288-UV 784

A three stage inoculum was prepared using a culture of LL-E33288-UV 784 (NRRL-18149). The inoculum media were of the following formulation:

| Ingredient | per/liter |
|---|---|
| Calcium carbonate | 4 g |
| Hodag® FD 82* | 1 ml |
| Dextrin | 24 g |
| Glucose | 5 g |
| Yeast extract | 5 g |
| Tryptone | 5 g |
| Beef extract | 3 g |
| Water qs | |

| | |
|---|---|
| *Hodag® FD 82 is a silicone antifoam agent. | |

The first stage consisted of 100 ml of the above sterile medium in a 500 ml flask incubated at 32°C and 200 rpm for 2 days. This first stage was used to inoculate a second stage consisting of 10 liters of the above sterile medium which was grown for 2 days at 32°C and 450 rpm in a small fermenter with a sterile air flow of one volume of air per volume of mash per minute (VVM). This second stage was used to inoculate a third stage consisting of 300 liters of the above sterile medium which was grown for 2 days at 32°C, 200-250 rpm and a sterile air flow of 0.67 VVM in a tank fermenter.

A 150 liter portion of this stage III inoculum was used to inoculate a sterile 1500 liter fermentation medium of the following composition:

| Ingredient | per/liter |
|---|---|
| Sucrose | 20.0 g |
| Ferrous sulfate heptahydrate | 0.1 g |
| Magnesium sulfate heptahydrate | 0.2 g |
| Peptone | 5.0 g |
| Molasses | 5.0 g |
| Potassium iodide* | 0.5 g |
| Calcium carbonate | 5.0 g |
| Hodag® FD 82 | 5.0 ml |
| Water qs | |

| | |
|---|---|
| *Potassium bromide may be substituted to produce the bromo analogs. | |

The fermentation was carried out at 30°C, a sterile air flow of 0.75 VVM, a back pressure of 8 psig and agitation by an impeller driven at 120 rpm for 5-6 days at which time the mash was harvested.

### Example 3

### Isolation of Crude LL-E33288α₃-I, LL-E33288β₁-I, LL-E33288γ₁-I and LL-E33288δ₁-I from a Fermentation of LL-E33288-UV-784 (NRRL-18149)

A 1500 liter portion of whole harvest mash containing 12.4 g of LL-E33288γ₁-I and 10.5 g of LL-E33288δ₁-I, which had been conducted essentially as described in Example 12, was mixed thoroughly with 1500 liters of ethyl acetate for 3 hours, then filter aid was added and the mixture filtered. The organic phase was separated, concentrated to 100 liters, adjusted to pH 6-7 with 2N sodium hydroxide and any aqueous phase discarded. The organic phase was further concentrated to 20 liters and any aqueous phase and interfacial fats removed. The organic phase was finally concentrated to a golden yellow syrup which was poured slowly into 7-8 times its volume of rapidly stirred hexane. The hexane insoluble gum, containing the LL-E33288 antibiotics, was collected, redissolved in 3 liters of ethyl acetate and dried over anhydrous sodium sulfate. The dried ethyl acetate solution was concentrated to a small volume and precipitated by the addition of ether and hexane, giving 53 g of crude LL-E33288 complex, containing 4.9 g of γ₁-I, 2.8 g of δ₁-I and small amounts of α₃-I and β₁-I.

### Example 4

### Separation of LL-E33288β₁-I, γ₁-I, δ₁-I and α₃-I

A 7.2 g portion of crude LL-E33288 complex from Example 13 was chromatographed on two Sepralyte C-18 (35-65 m) columns (2.5 x 23 cm), eluting with acetonitrile:0.2 M aqueous ammonium acetate (45:55) at 12 ml/minute, collecting sixty 24 ml fractions from each column. Each fraction was analyzed by TLC (EM silica gel 60F₂₅₄ pre-coated aluminum sheets, 3% isopropanol in ethyl acetate saturated with 0.1 M KH₂PO₄ elution, detected with UV₂₅₄ₙₘ quenching and bioautography via the BIA) and those containing γ₁-I were pooled and concentrated on a rotary evaporator to remove acetonitrile. The aqueous mixture was extracted twice with equal volumes of ethyl acetate and the ethyl acetate solution dried over anhydrous sodium sulfate, concentrated and precipitated by addition of hexane to yield 504 mg of partially purified LL-E33288γ₁-I (60% pure) containing β₁-I.

Fractions containing α₃-I and δ₁-I eluting off the column ahead of γ₁-I, were pooled separately and worked up to yield 812 mg of partially purified α₃-I (12% pure) and 1336 mg (22% δ₁-I, 20% γ₁-I) of a partially purified mixture of δ₁-I and γ₁-I.

### Example 5

### Purification of LL-E332288γ₁-I

A 309 mg portion of partially purified γ₁-I (66% pure) was chromatographed on a Sephadex® LH-20 (hydroxy propylated dextran) column (1.5 x 90 cm) equilibrated with hexane:dichloromethane:ethanol (2:1:1). The column was eluted with the same solvent system at 1.5 ml/minute and twenty-five 20 ml fractions were collected and analyzed as before. Fractions containing pure γ₁-I were pooled and concentrated as before to a light yellow residue. This residue was redissolved in ethyl acetate and precipitated by the addition of hexane to yield 194 mg of pure LL-E33288γ₁-I.

### Example 6

### Purification of LL-E332288β₁-I

A 1.05 g portion of partially purified γ₁-I containing β₁-I (61% γ₁-I, 10% β₁-I) was chromatographed on a Woelm silica (32-63 µ) column (1.5 x 45 cm) packed and equilibrated with ethyl acetate. The column was eluted with ethyl acetate at 3.6 ml/minute for one hour, then the eluent was changed to ethyl acetate:methanol (97:3) and the elution was continued for 2 hours. Fractions of 18 ml were collected during the entire elution. Each fraction was analyzed as before and those containing β₁-I were pooled and worked up to yield 56 mg of 86% pure LL-E33288β₁-I.

Fractions containing γ₁-I were also worked up to yield 385 mg of 74% pure LL-E33288γ₁-I.

### Example 7

### Purification of LL-E33288δ₁-I

A partially purified mixture of δ₁-I and γ₁-I (1.8 g, containing 648 mg of γ₁-I and 540 mg of δ₁-I) was chromatographed on a Woelm silica (32-63 µ) column (1.5 x 45 cm) packed and equilibrated with ethyl acetate. The column was eluted with ethyl acetate at 3 ml/minute for one hour, then the eluent was changed to ethyl acetate:methanol (97:3) and the elution was continued for 2 hours. Fractions of 15 ml were collected during the entire elution. Each fraction was analyzed as before and those containing pure δ₁-I were pooled and worked up to yield 367 mg of pure LL-E33288δ₁-I.

Fractions containing γ₁-I were also worked up to yield 574 mg of 65% pure LL-E33288γ₁-I.

### Example 8

### Purification of LL-E33288α₃-I

A partially purified sample of α₃-I (1.8 g, containing 310 mg of α₃-I) was chromatographed on a Sephadex® LH-20 column (1.5 x 90 cm) equilibrated with hexane:dichloromethane:ethanol (2:1:1). The column was eluted with the same solvent system at 4 ml/minute and forty-five 20 ml fractions were collected and analyzed as before. Fractions containing pure α₃-I were pooled and concentrated as before to a light yellow residue which was redissolved in ethyl acetate and precipitated by the addition of hexane to yield 289 mg of pure LL-E33288α₃-I.

### Example 9

### Preparation of LL-E33288α₂-I from LL-E33288γ₁-I

A 300 mg portion of partially purified γ₁-I (60% pure) was dissolved in 60 ml of 2% hydrogen chloride in methanol and the solution was allowed to remain at room temperature for 6 hours. The reaction mixture was then neutralized by the addition of a saturated methanolic solution of potassium carbonate. The precipitated potassium chloride was filtered off and the solution was concentrated to dryness. The ethyl acetate soluble portion of the residue was concentrated and precipitated from hexane to yield 135 mg of crude LL-E33288α₂-I.

This crude α₂-I was purified by chromatography on a Bio-Sil® (20-40 µ) column (1.5 x 20 cm) eluting with dichloromethane:methanol (96:4) to give 34 mg of analytically pure LL-E33288α₂-I. The small amounts of LL-E33288α₂-I isolated previously from the fermentation of NRRL-15839 was identified with the LL-E33288α₂-I prepared as described in this example by TLC and HPLC analyses.

Degradation products, termed pseudaglycones, of LL-E33288, BBM-1675, FR-900405, FR-900406, PD 114759, PD 115028, CL-1577A, CL-1577B, CL-1577D, CL-1577E and CL-1724 antibiotics/antitumor agents are disclosed and described.

Certain other antibiotics are pertinent to this invention, namely:
1) Esperamicin BBM-1675, a novel class of potent antitumor antibiotics. I. Physico-chemical data and partial structure. M. Konishi, et al., J. Antibiotics, 38, 1605 (1985). A new antitumor antibiotic complex. M. Konishi, et al., UK Patent application GB 2,141,425A, May 15, 1984.
2) New antitumor antibiotics, FR-900405 and FR-900406. I. Taxonomy of the producing strain. M. Iwami, et al., J. Antibiotics, 38, 835 (1985). New antitumor antibiotics FR-900405 and FR-900406. II. Production, isolation, characterization and antitumor activity. S. Kiyoto, et al., J. Antibiotics, 38, 840 (1985).
3) PD 114759 and PD 115028, novel antitumor antibiotics with phenomenal potency. I. Isolation and characterization. R. H. Bunge, et al., J. Antibiotics, 37, 1566 (1984). Biological and biochemical activities of the novel antitumor antibiotic PD 114759 and related derivatives. D. W. Fry, et al., Investigational New Drugs, 4, 3 (1986).
4) New antibiotic complex CL-1577A and CL-1577B produced by *Streptomyces* sp. ATCC 39363. European Patent application 0,132,082,A2.
5) CL-1577D and CL-1577E Antibiotic antitumor compounds, their production and use. U. S. Patent 4,539,203.
6) CL-1724 Antibiotic compounds, their production and use. U. S. Patent 4,554,162.

All of the information regarding BBM-1675, FR-900405, FR-900406, PD 114759, PD 115028, CL-1577A, CL-1577B, CL-1577D, CL-1577E and CL-1724 contained in the above citations is incorporated herein by reference.

### EXAMPLE 13

### Preparation of Dihydro-LL-E33288γ₁-I

A 10 ml portion of methyl iodide was added to a solution of 126 mg of LL-E33288γ₁-I in 25 ml of ethanol and the mixture was cooled in an ice-water bath. To this was added 12 ml of a 0.1M ethanolic solution of sodium borohydride, in 2 ml portions. When the reaction was complete, the borate complex was decomposed by the addition of 1.2 ml of a 4M ethanolic solution of acetic acid. The reaction mixture was then concentrated to a golden yellow residue which was redissolved in ethyl acetate and then reconcentrated to dryness. This residue was redissolved in ethyl acetate, the insolubles filtered off, the filtrate concentrated to a small volume and precipitated by the addition of hexane. The 301 mg of crude dihydro-LL-E33288γ₁-I was purified by chromatography on a Bio-Sil A (20-44µ) column, eluting with dichloromethane:methanol (92:8), giving 57 mg of pure dihydro-LL-E33288γ₁-I as a 30:70 mixture of two regio isomers.

### Example 14

### Preparation of Dihydro-LL-E33288-pseudoaglycone

A 10 ml portion of methyl iodide was added to a solution of 112 mg of LL-E33288-pseudoaglycone in 25 ml of ethanol and this mixture was cooled in an ice-water bath. To this was added 12 ml of a 0.025M ethanolic sodium borohydride in 2 ml portions. When the reaction was complete, the borate complex was decomposed by the addition of 1.2 ml of a 1M ethanolic solution of acetic acid. The reaction mixture was then concentrated to a golden yellow residue, redissolved in ethyl acetate and then reconcentrated to dryness. This residue was redissolved in ethyl acetate, the insolubles filtered off, the filtrate concentrated to a small volume and precipitated by the addition of hexane. The 128 mg of crude dihydro-LL-E33288-pseudoaglycone was purified by chromatography on a Bio-Sil A (20-44µ) column, eluting with dichloromethane:methanol (97:3), giving 42 mg of pure dihydro-LL-E33288-pseudoaglycone.

## Claims (Claims for the following Contracting State(s): GR and ES)

1. A method for preparing a compound selected from:
the dihydro derivative of the iodinated pseudoaglycone of LL-E33288 of the formula: 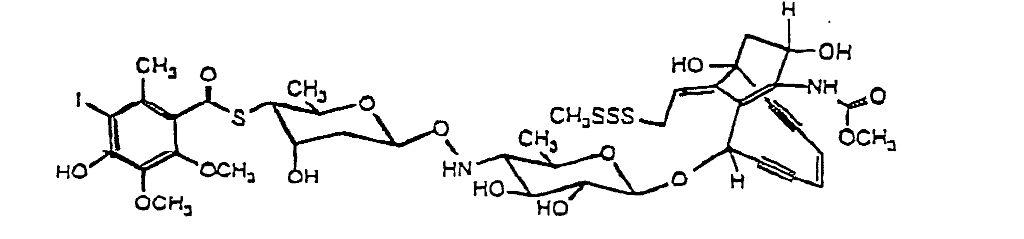
the dihydro derivative of the brominated pseudoaglycone of LL-E33288 of the formula: 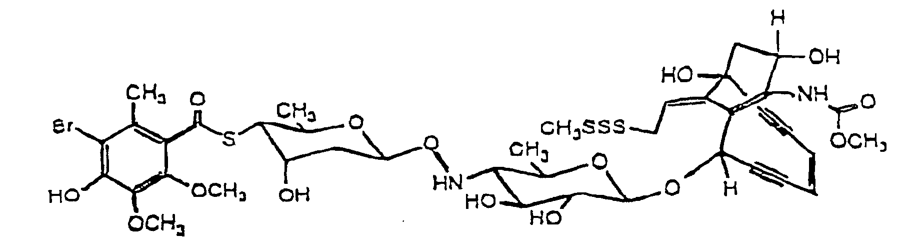
the dihydro derivative of LL-E33288α₂-Br of the formula: 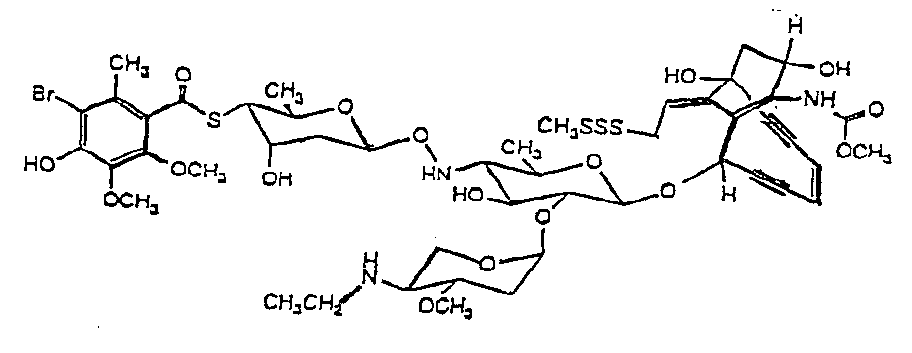
the dihydro derivative of LL-E33288α₃-Br of the formula: 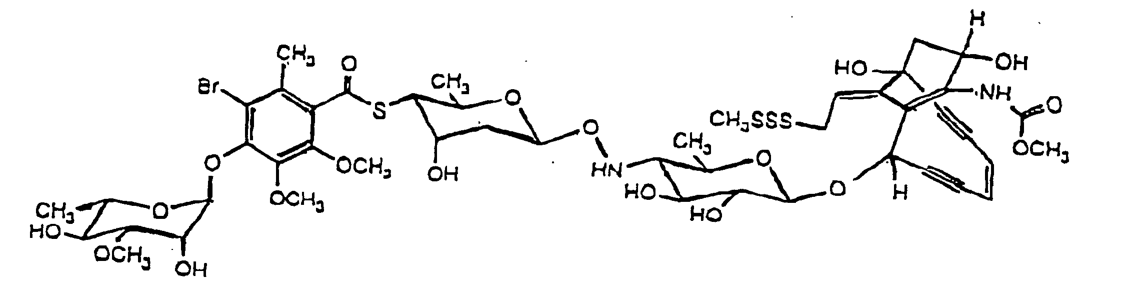
the dihydro derivative of LL-E33288β₁-Br of the formula: 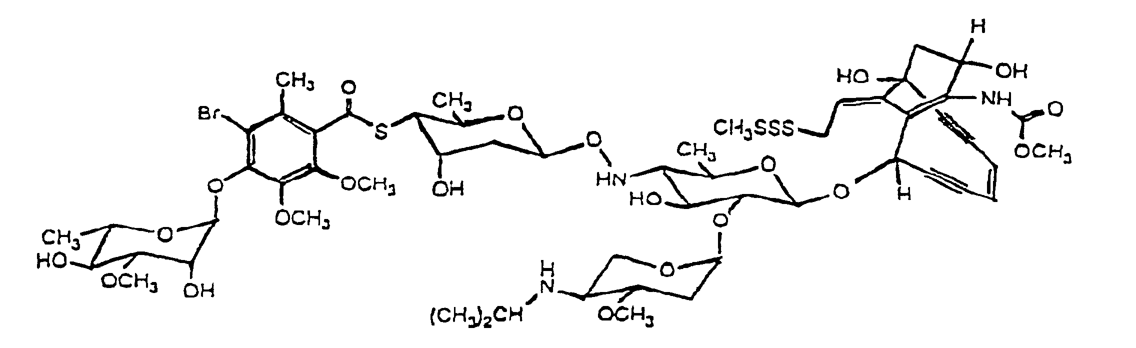
the dihydro derivative of LL-E33288γ₁-Br of the formula: 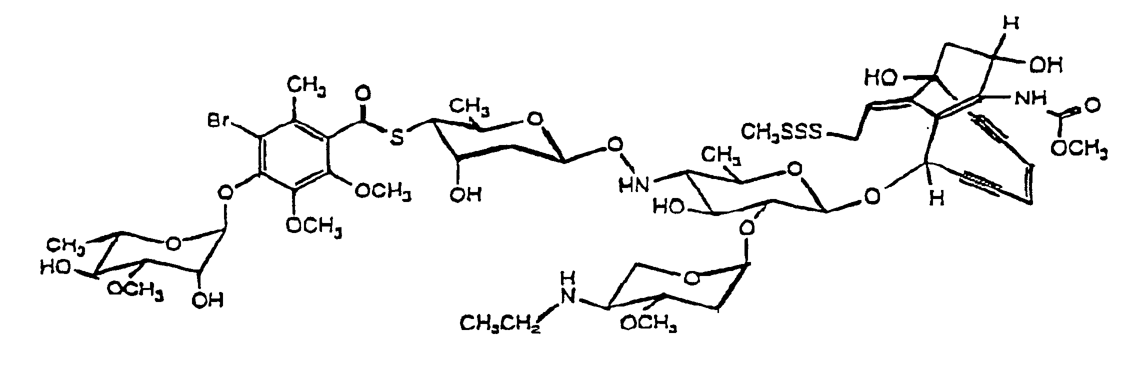
the dihydro derivative of LL-E33288α₂-I of the formula: 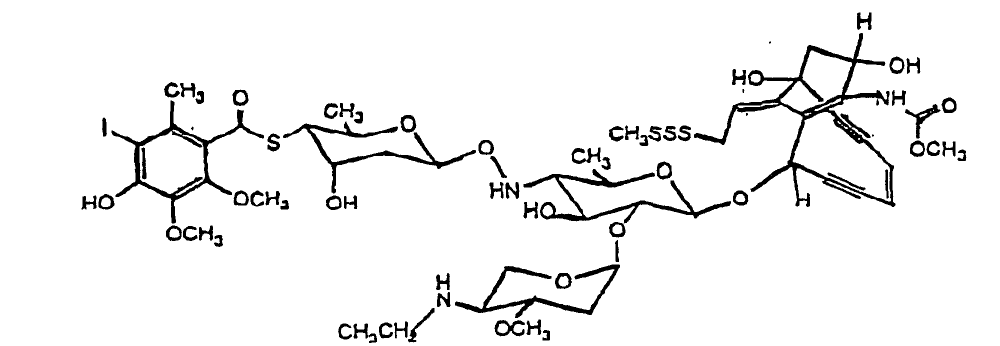
the dihydro derivative of LL-E33288α₃-I of the formula: 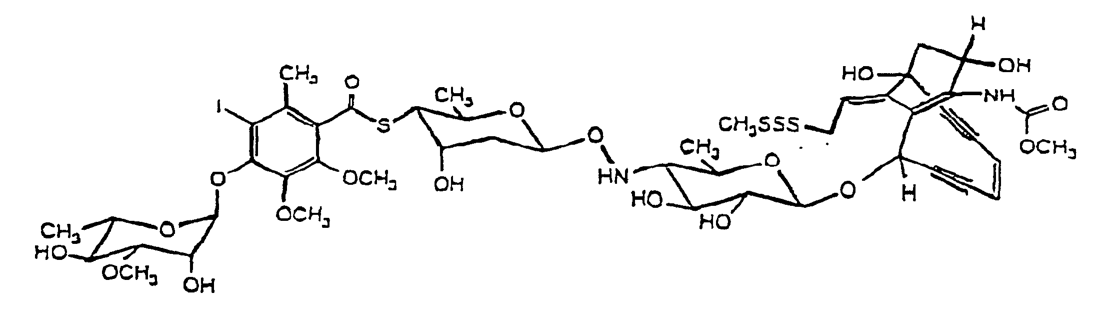
the dihydro derivative of LL-E33288β₁-I of the formula: 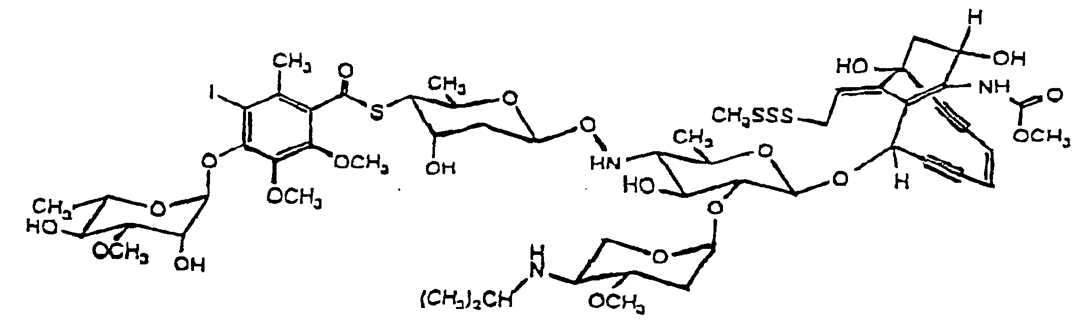
the dihydro-LL-E33288 γ₁-I of the formula: 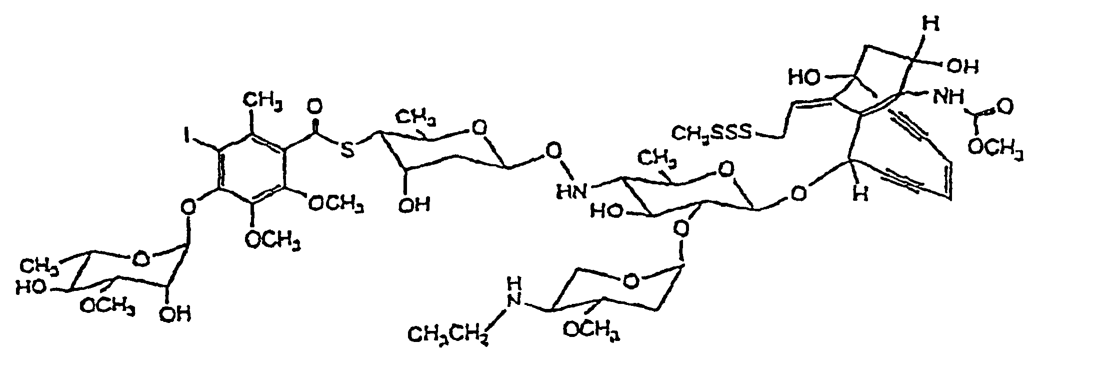
or the dihydro derivative of LL-E33288 δ₁- of the formula: 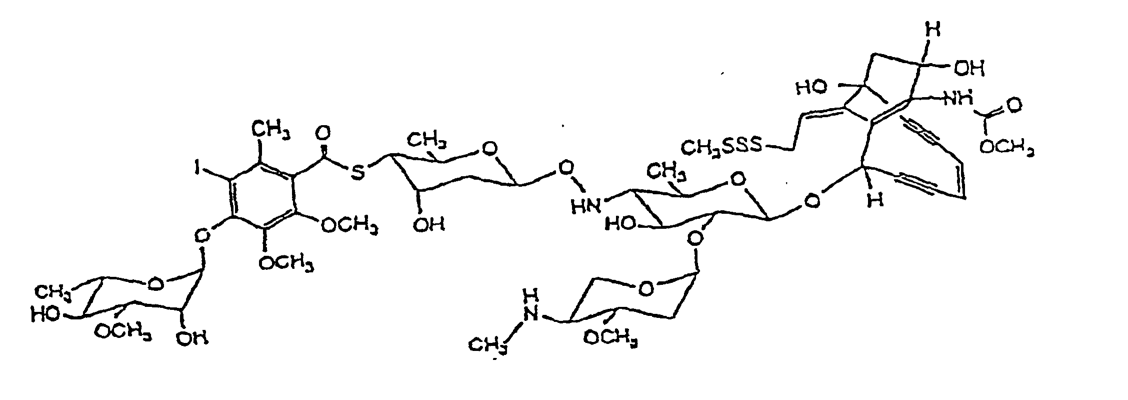
said method comprising the step of:
adding an alkyl iodide to an ethanolic solution of the appropriate antibiotic selected from iodinated pseudoaglycone of LL-E33288, brominated pseudoaglycone of LL-E33288, LL-E33288 α₂-Br, LL-E33288 α₃-Br, LL-E33288 β₁-Br, LL E33288 γ₁-Br, LL-E33288 α₂-I, LL-E33288 α ₃-I, E33288 γ1 or LL-E33288 δ₁, cooling the solution to ice-bath temperature, adding an ethanolic solution of sodium borohydride, decomposing the borate complex by the addition of acetic acid, precipitating from an ethyl acetate solution by the addition of hexane and purifying by chromatography.

2. A method as claimed in claim 1 wherein the antitumor antibiotic produced is designated dihydro-LL-E33288-pseudoaglycone and has the following physico-chemical characteristics:
a) molecular weight 1052 (determined by FAB-MS);
b) ultraviolet absorption spectrum as shown in Figure I (methanol);
c) infrared absorption spectrum as shown in Figure II (KBr disc);
d) proton magnetic resonance spectrum as shown in Figure III (300MHz, CDCl₃); and
e) carbon-13 magnetic resonance spectrum as shown in Figure IV (75 MHz, CDCl₃) .

3. A process for preparing a LL-E33288 antitumor antibiotic which comprises aerobically fermenting a liquid medium containing assimilable sources of carbon, nitrogen, iodine and inorganic salts, which medium has been inoculated with a viable culture of the organism **Micromonosporal echinospora** ssp. **calichensis** mutant NRRL-18149 or NRRL-15839 or mutants of NRRL-18149, until substantial antibiotic activity is imparted; maintaining said fermentation culture at a temperature of about 24-32°C for a period of approximately 90-200 hours; harvesting the mash and recovering the antibiotic, adding an alkyl iodide to an ethanolic solution of antibiotic; cooling the solution to ice-bath temperature; adding an ethanolic solution of sodium borohydride; decomposing the borate complex by the addition of acetic acid, precipitating from an ethyl acetate solution by the addition of hexane and purifying by chromatography.

4. A compound as defined in any one of claims 1 to 3 for use in treating bacterial infections in warm-blooded animals.

5. A compound as defined in any one of claims 1 to 3 for use in inhibiting the growth of tumors in a mammal.

6. A compound as defined in any one of claims 1 to 3 for use in regressing leukemia in a mammal.

7. The use of a compound as defined in any one of claims 1 to 3 in the manufacture of a medicament for treating bacterial infections in warm-blooded animals.

8. The use of a compound as defined in any one of claims 1 to 3 in the manufacture of a medicament for inhibiting the growth of tumors in a mammal.

9. The use of a compound as claimed in any one of claims 1 to 3 in the manufacture of a medicament for regressing leukemia in a mammal.

10. A method for preparing a composition of matter in unit dosage form comprising combining an antibacterially effective amount of a compound as defined in any one of claim 1 to 3 with a suitable carrier.

## Claims (Claims for the following Contracting State(s): AT, BE, CH, LI, DE, FR GB, IT , NL, SE)

1. The dihydro derivative of the iodinated pseudoaglycone of LL-E33288 of the formula:

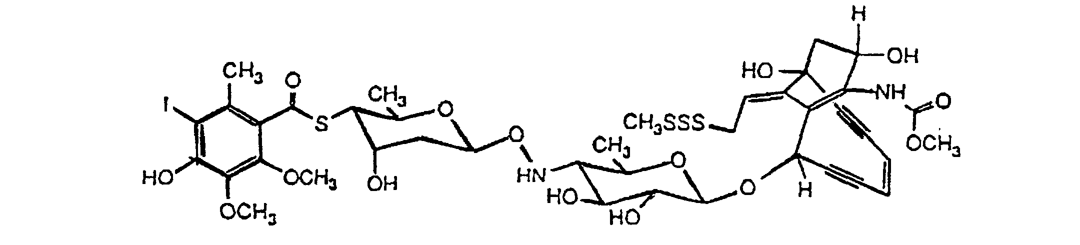

2. The dihydro derivative of the brominated pseudoaglycone of LL-E33288 of the formula:

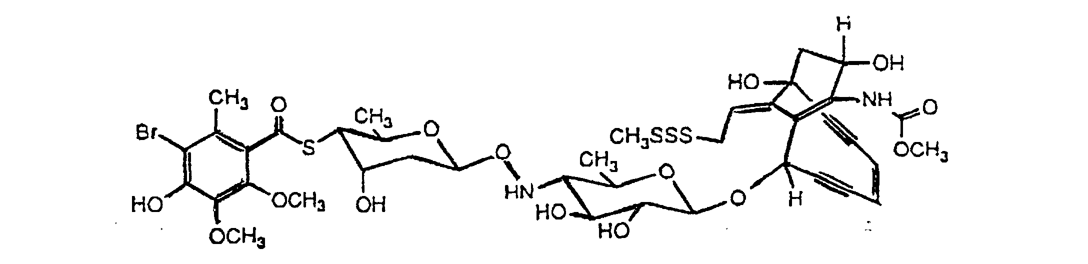

3. The dihydro derivative of LL-E33288α₂-Br of the formula: 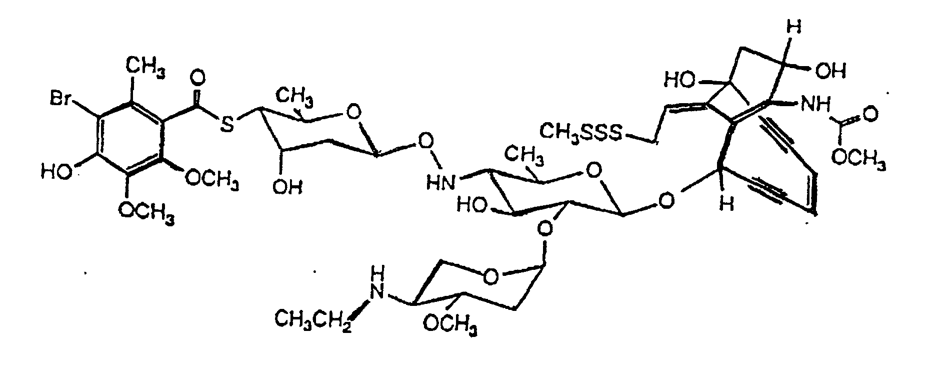
the dihydro derivative of LL-E33288α₃-Br of the formula: 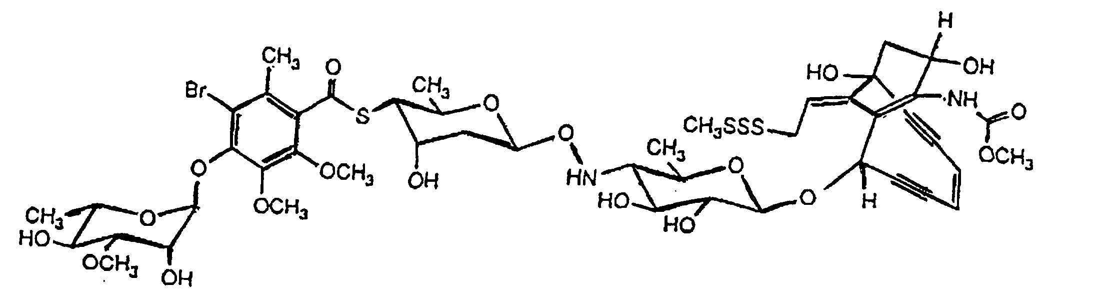
the dihydro derivative of LL-E33288β₁-Br of the formula: 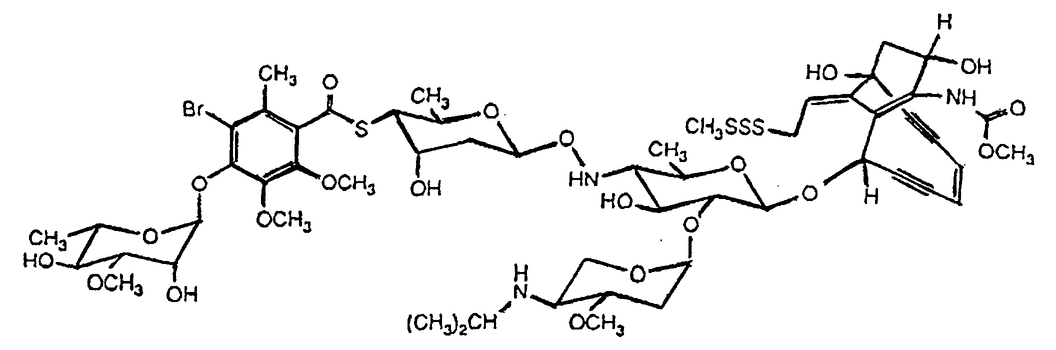
the dihydro derivative of LL-E33288γ₁-Br of the formula: 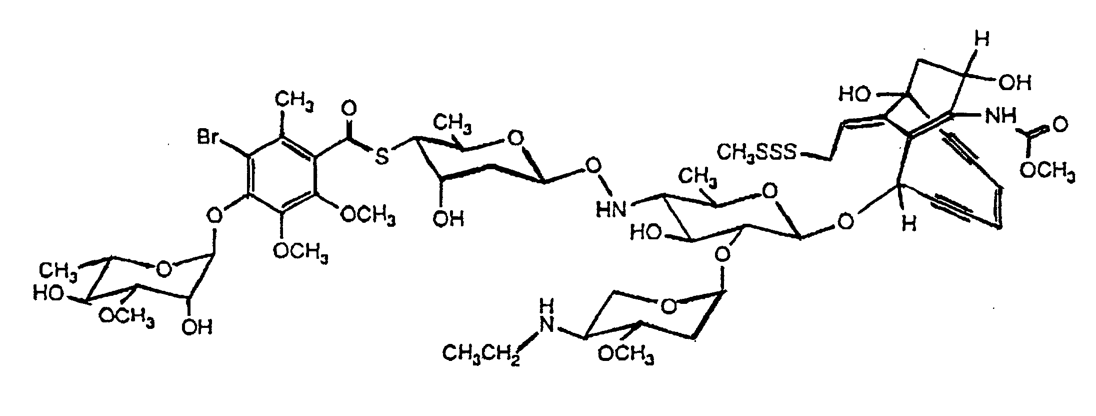
the dihydro derivative of LL-E33288α₂-I of the formula: 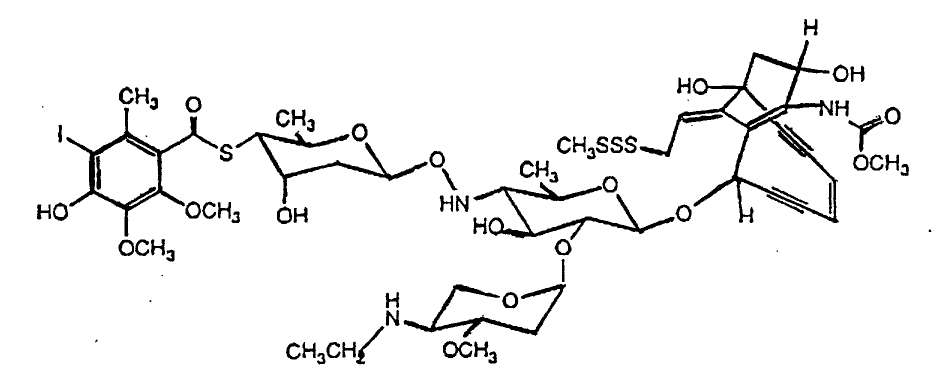
the dihydro derivative of LL-E33288α₃-I of the formula: 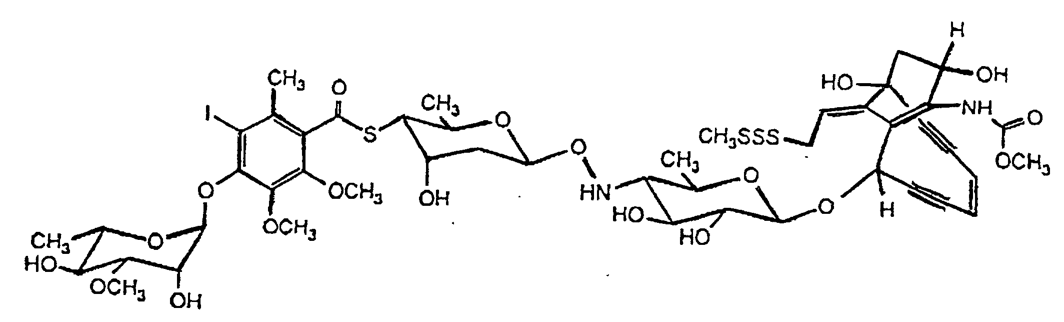
the dihydro derivative of LL-E33288β₁-I of the formula: 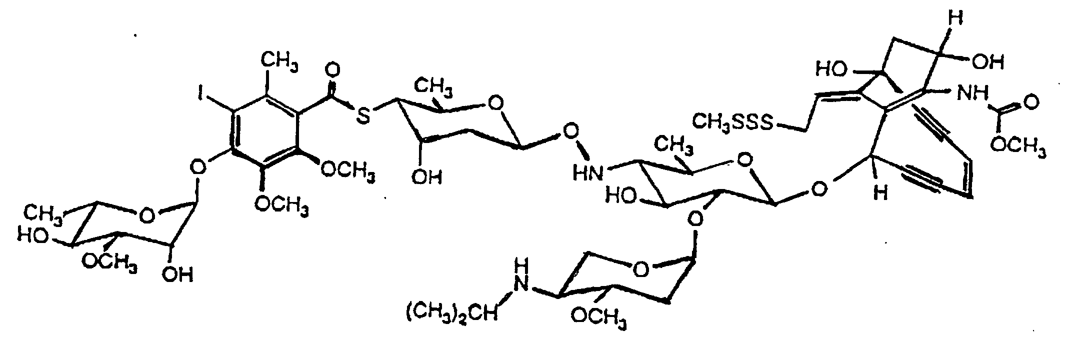
the dihydro derivative of LL-E33288γ₁-I of the formula:

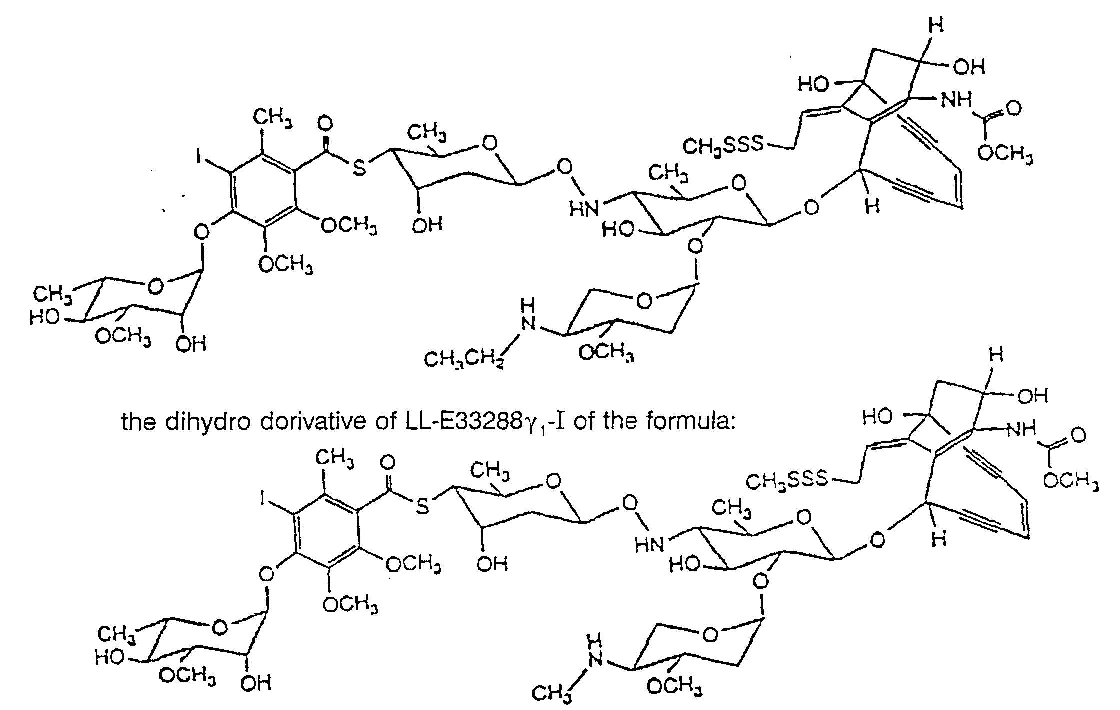

4. An LL-E33288 antitumor antibiotic obtainable inter alia by:
aerobically fermenting a liquid medium containing assimilable sources of carbon, nitrogen, iodine and inorganic salts, which medium has been inoculated with a viable culture of the organism **Micromonosporal echinospora** ssp. **calichensis** mutant NRRL-18149 or NRRL-15839 or mutants of NRRL-18149, until substantial antibiotic activity is imparted;
maintaining said fermentation culture at a temperature of about 24-32°C for a period of approximately 90-200 hours; harvesting the mash and recovering the antibiotic; adding an alkyl iodide to an ethanolic solution of the antibiotic; cooling the solution to ice-bath temperature;
adding an ethanolic solution of sodiuxm borohydride; decomposing the borate complex by the addition of acetic acid, precipitating from an ethyl acetate solution by the addition of hexane and purifying by chromatography.

5. An antitumor antibiotic as claimed in Claim 4 designated dihydro-LL-E33288-pseudoaglycone having the following physico-chemical characteristics:
a) molecular weight 1052 (determined by FAB-MS);
b) ultraviolet absorption spectrum as shown in Figure I (methanol);
c) infrared absorption spectrum as shown in Figure II (KBr disc);
d) proton magnetic resonance spectrum as shown in Figure III (300 MHz, CDCl₃); and
e) carbon-13 magnetic resonance spectrum as shown in Figure IV (75 MHz, CDCl₃).

6. A compound as claimed in any one of the preceding claims for use in treating bacterial infections in warm-blooded animals.

7. A compound as claimed in any one of claims 1 to 5 for use in inhibiting the growth of tumors in a mammal.

8. A compound as claimed in any one of claims 1 to 5 for use in regressing leukemia in a mammal.

9. The use of a compound as claimed in any one of claims 1 to 5 in the manufacture of a medicament for treating bacterial infections in warm-blooded animals.

10. The use of a compound as claimed in any one of claims 1 to 5 in the manufacture of a medicament for inhibiting the growth of tumors in a mammal.

11. The use of a compound as claimed in any one of claims 1 to 5 in the manufacture of a medicament for regressing leukemia in a mammal.

12. A process for producing the dihydro derivative of the iodinated pseudoaglycone of LL-E33288, dihydro derivative of the brominated pseudoaglycone of LL-E33288, dihydro-LL-E33288 α₂-Br, dihydro-LL-E33288 α₃-Br, dihydro-LL-E33288 β₁-Br, dihydro-LL-E33288 γ₁-Br, dihydro-LL-E332B8 α₂-I, dihydro-LL-E33288 α₃-I, dihydro-LL-E33288 β₁-I, dihydro-LL-E33288 γ₁-I or dihydro-LL-E33288 δ₁-I as defined in any one of claims 1 to 5 which comprises adding an alkyl iodide to an ethanolic solution of the antibiotic, cooling the solution to ice-bath temperature, adding an ethanolic solution of sodium borohydride, decomposing the borate complex by the addition of acetic acid, precipitating from an ethyl acetate solution by the addition of hexane and purifying by chromatography.

13. A composition of matter in unit dosage form comprising an antibacterially effective amount of a compound as claimed in any one of claims 1 to 5.

## Patentansprüche (Patentansprüche für folgende(n) Vertragsstaat(en): GR, ES)

1. Dihydroderivat des iodierten Pseudoaglykons von LL-E33288 mit der Formel:

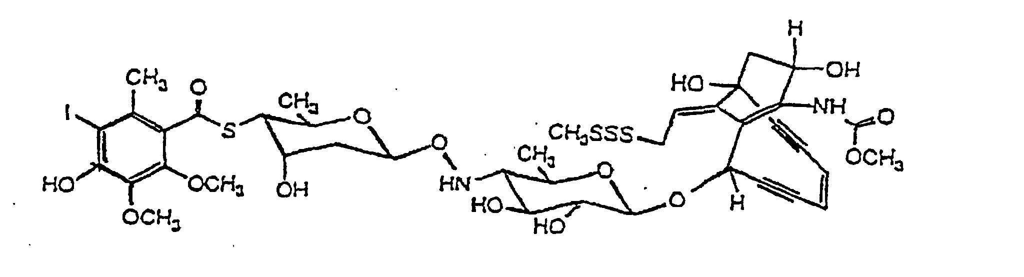

2. Dihydroderivat des bromierten Pseudoaglykons von LL-E33288 mit der Formel:

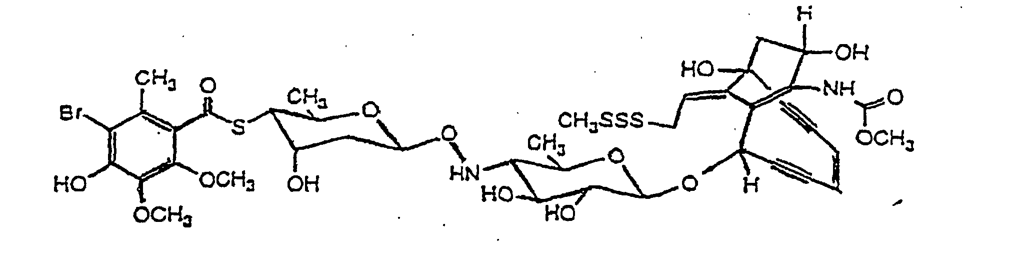

3. Dihydroderivat von LL-E33288α₂-Br mit der Formel: 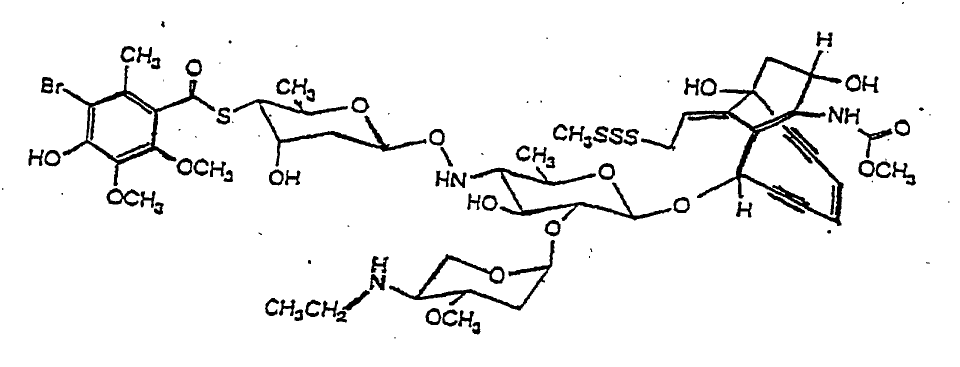
das Dihydroderivat von LL-E33288α₁-Br mit der Formel : 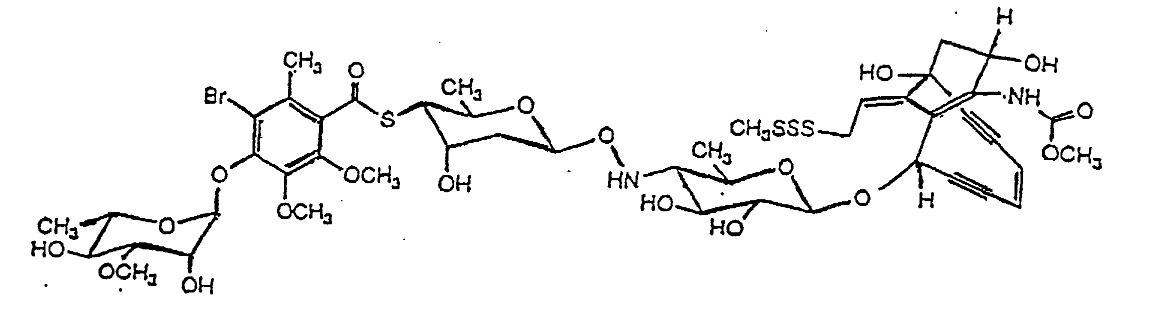
das Dihydroderivat von LL-E33288β₁-Br mit der Formel: 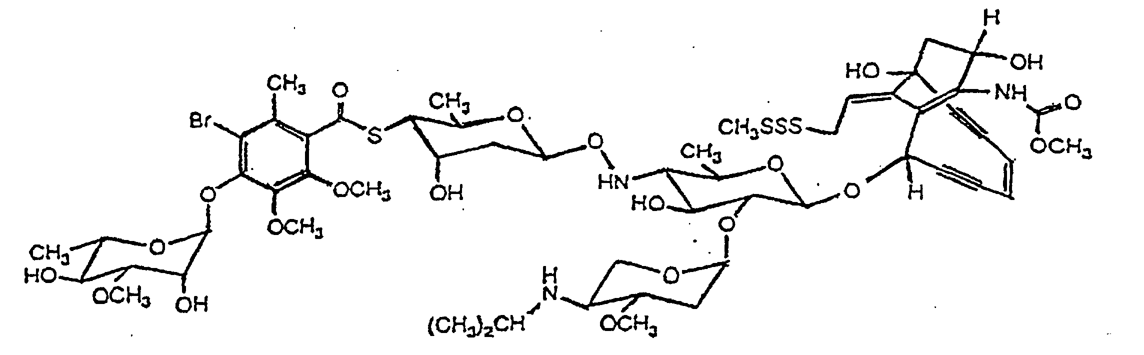
das Dihydroderivat von LL-E33288γ₁-Br mit der Formel: 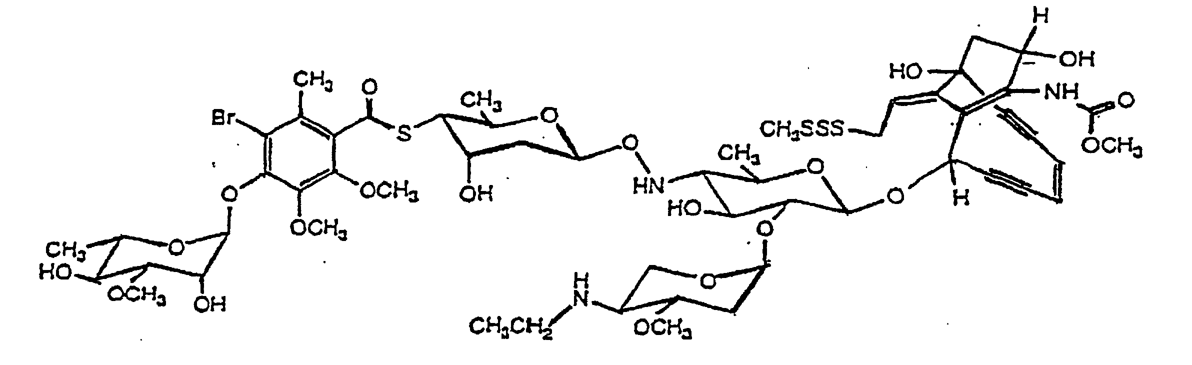
das Dihydroderivat von LL-E33288α₂-I mit der Formel: 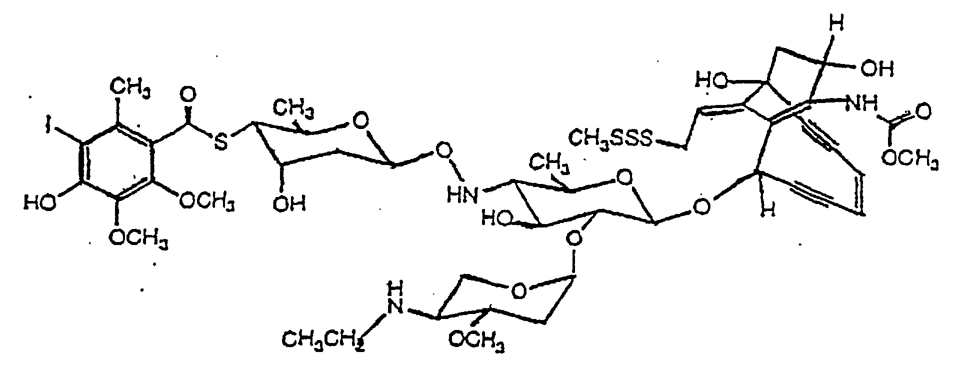
das Dihydroderivat von LL-E33288α₃-I mit der Formel: 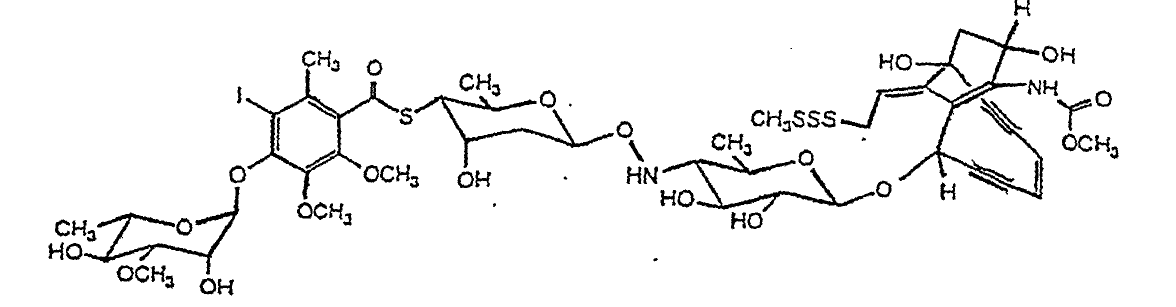
das Dihydroderivat von LL-E33288β₁-I mit der Formel: 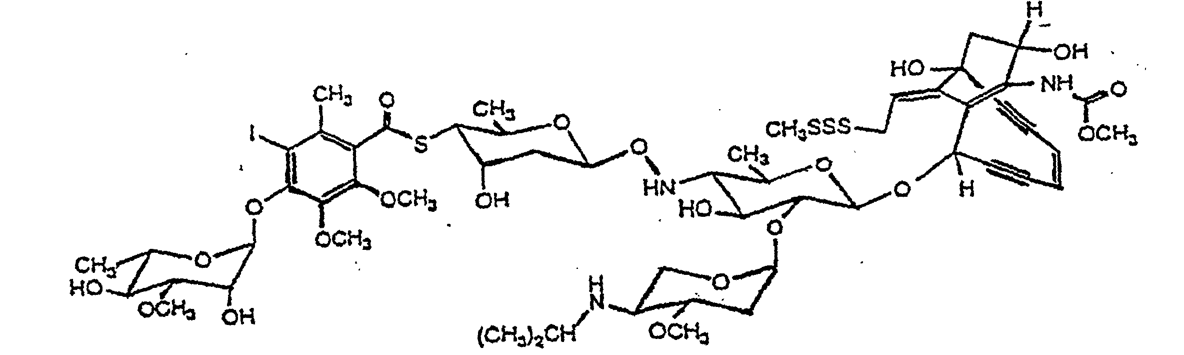
das Dihydroderivat von LL-E33288γ₁-I mit der Formel: 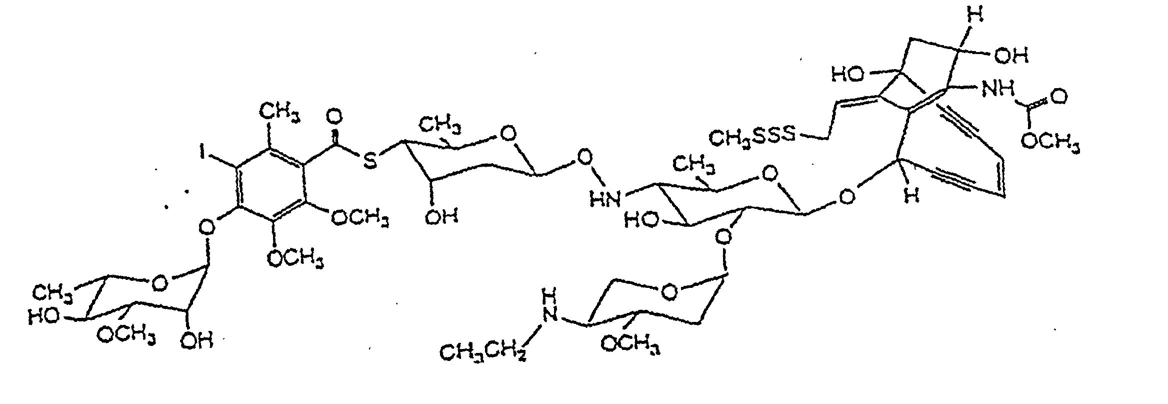
das Dihydroderivat von LL-E33288γ₁-I mit der Formel:

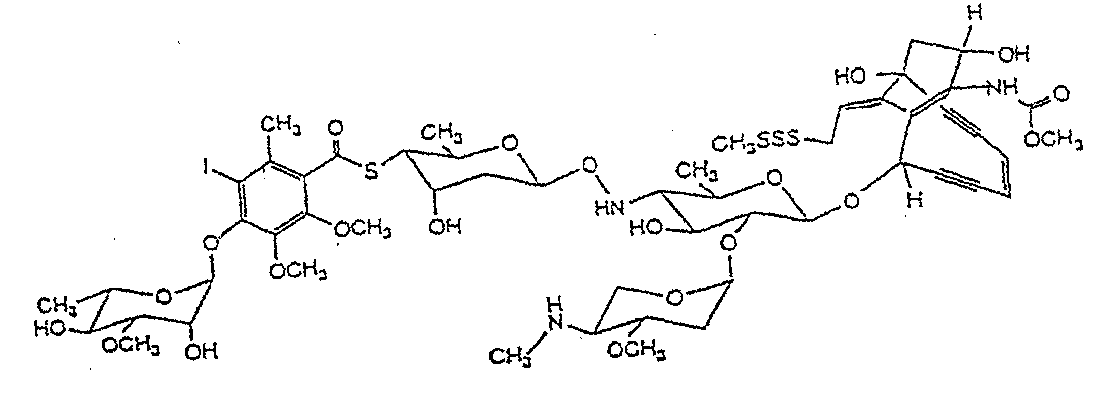

4. LL-E33288-Antitumorantibiotikum, das erhalten werden kann u.a. durch:
aerobes Fermentieren eines flüssigen Mediums, das assimilierbare Quellen von Kohlenstoff, Stickstoff, Iod und anorganischen Salzen enthält, welches Medium mit einer lebensfähigen Kultur des Organismus Micromonosporal echinospora ssp. calichensis von Mutanten NRRL-18149 oder NRRL-15839 oder Mutanten von NRRL-18149 geimpft wurde, bis eine wesentliche antibiotische Aktivität vermittelt ist;
Halten der Fermentationskultur bei einer Temperatur von etwa 24 - 32°C für eine Zeitspanne von ungefähr 90 - 200 Stunden; Ernten der Maische und Rückgewinnen des Antibiotikums; Addieren eines Alkyliodids zu einer Ethanollösung des Antibiotikums; Kühlen der Lösung auf Eisbadtemperatur; Addieren einer Ethanollösung von Natriumborhydrid; Aufschließen des Boratkomplexes durch die Addition von Essigsäure, Ausfällen aus einer Ethylacetatlösung durch die Addition von Hexan und Reinigen durch Chromatographie.

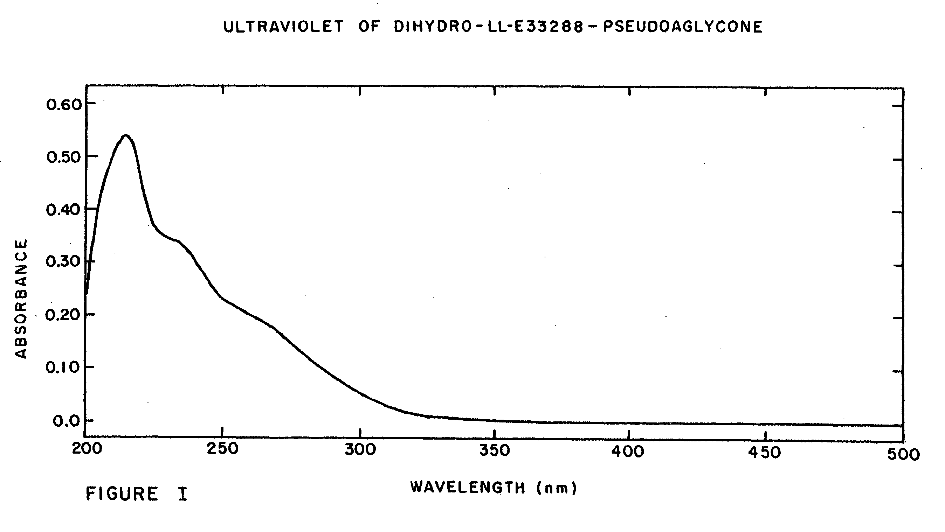

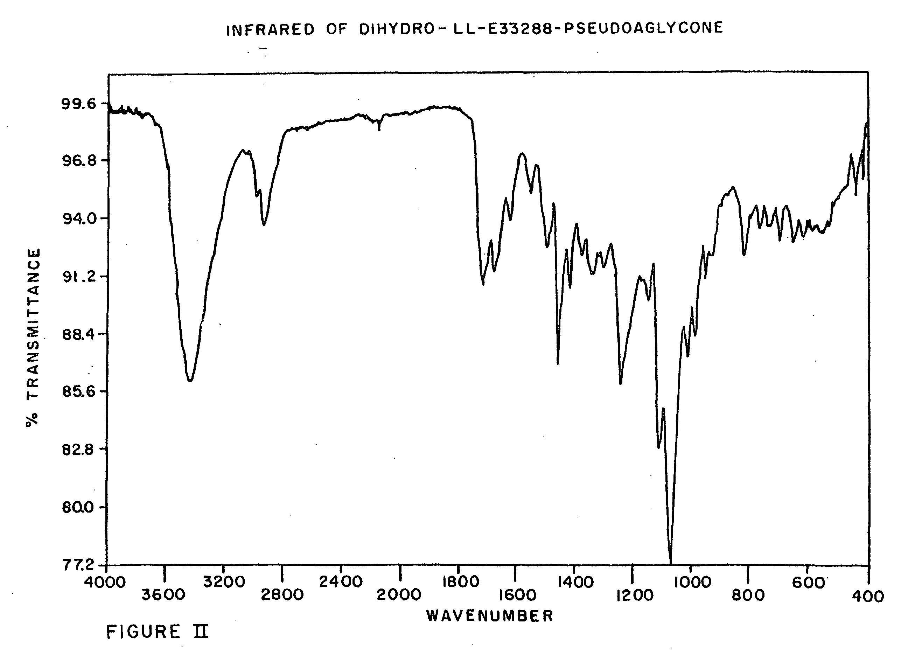

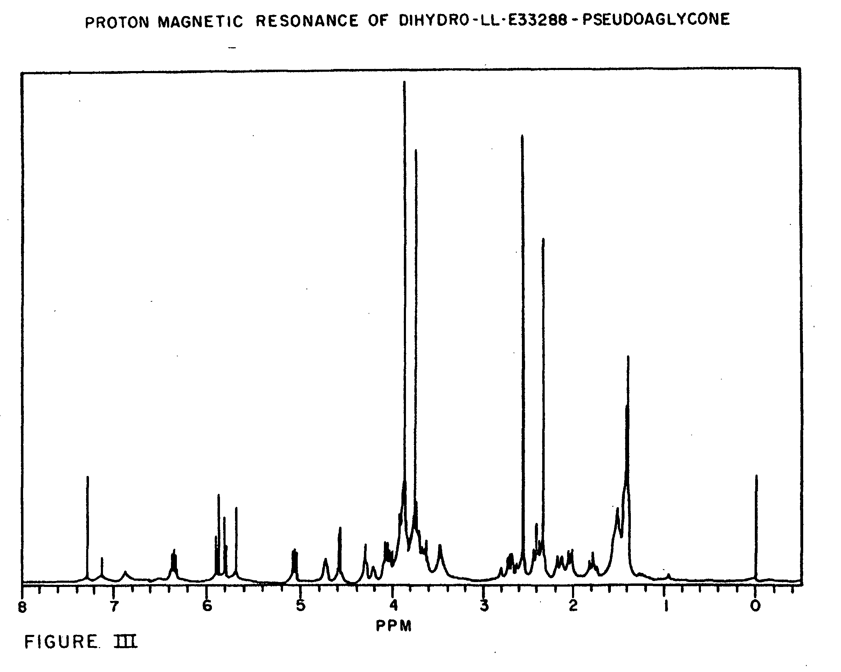

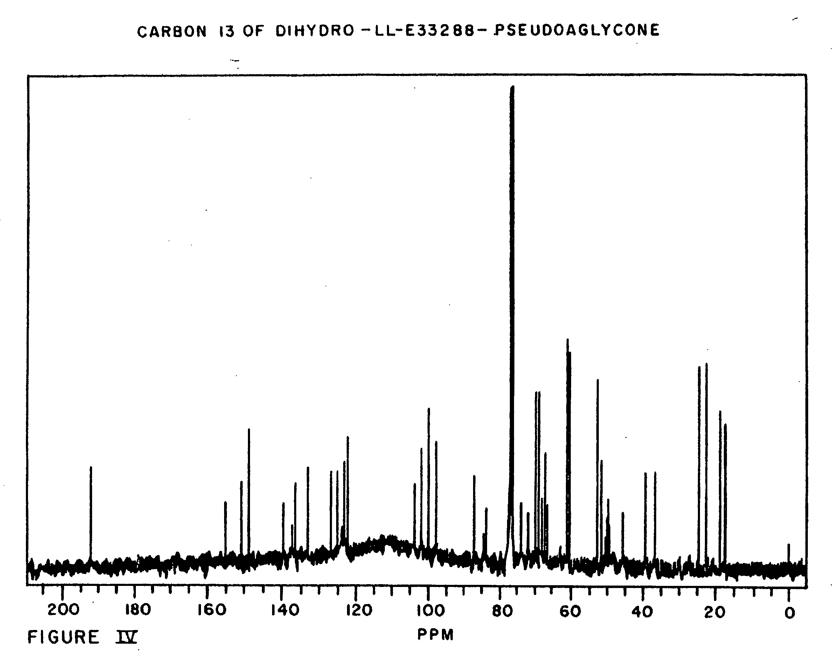

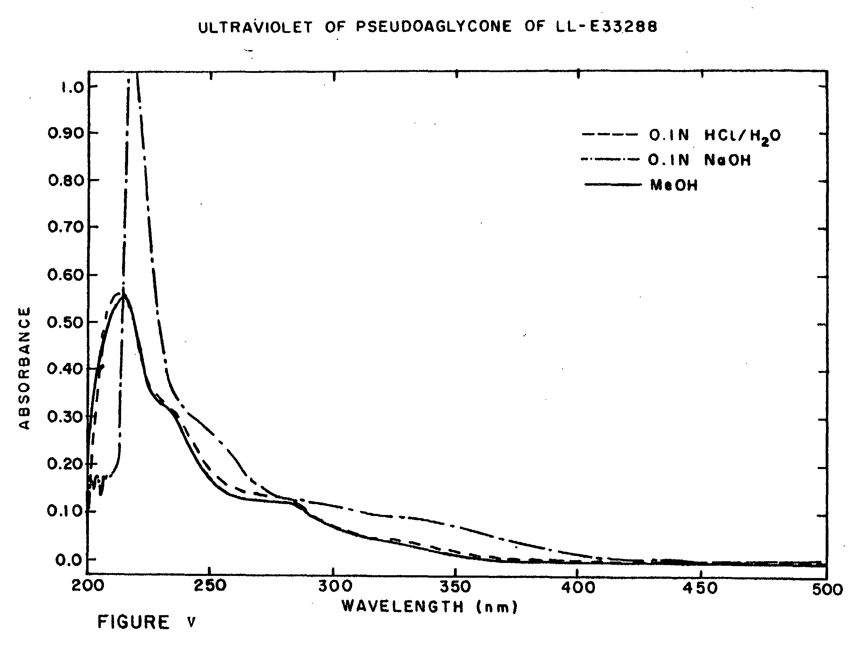

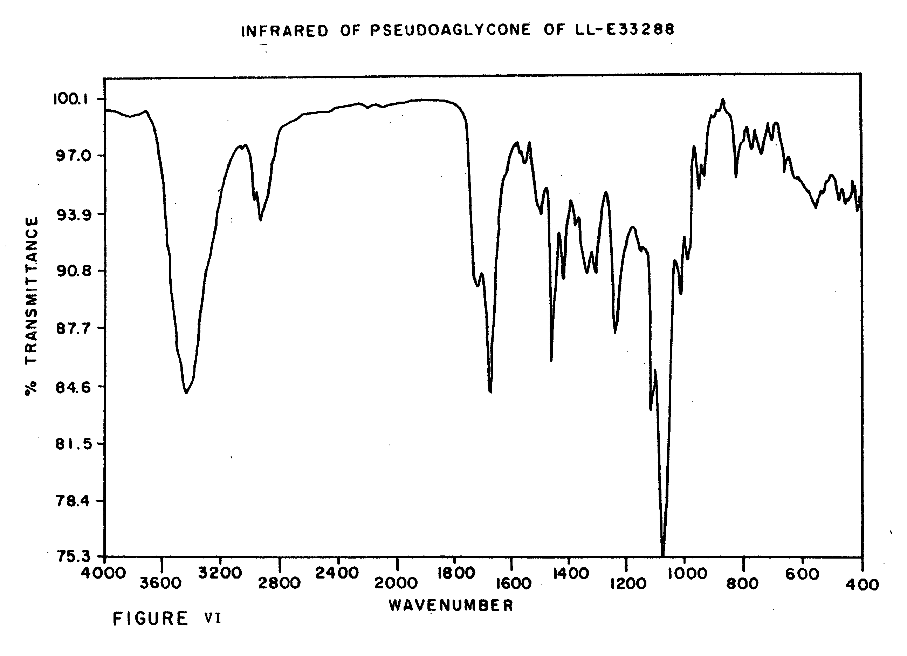

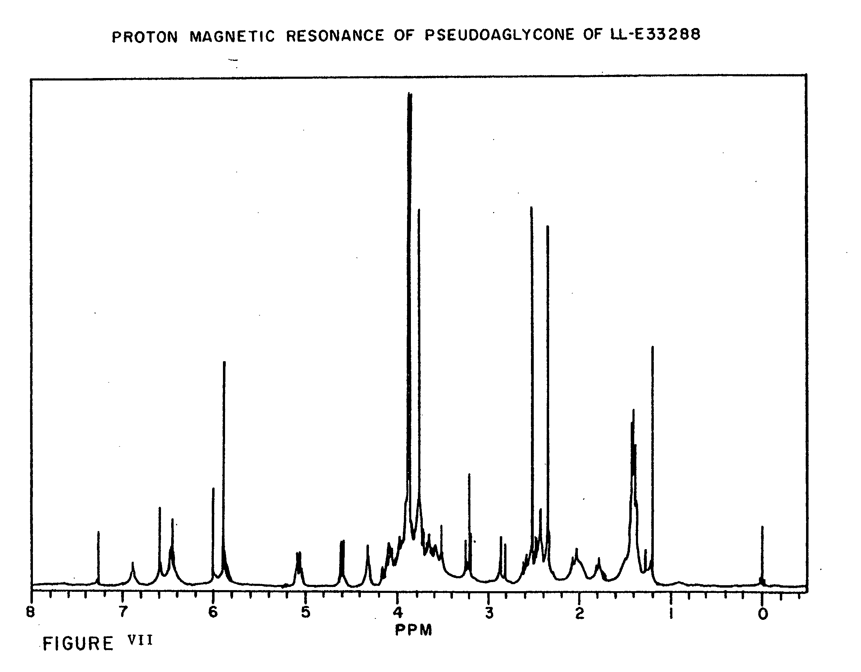

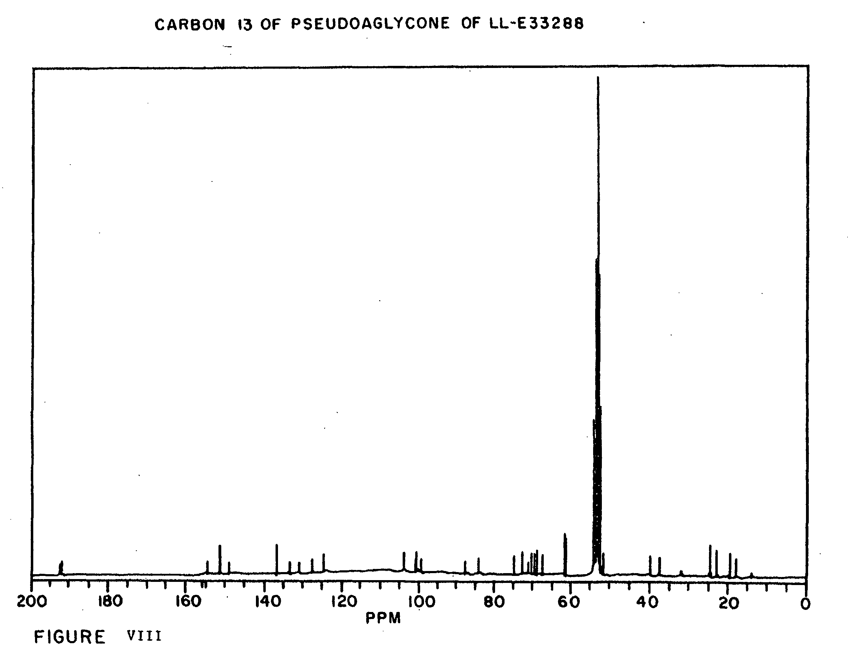

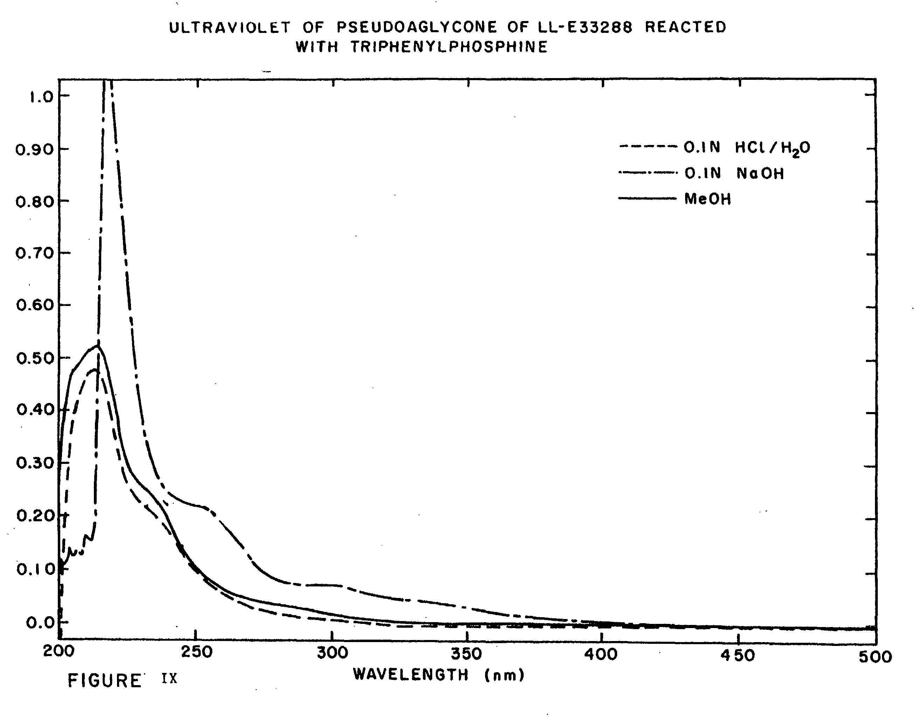

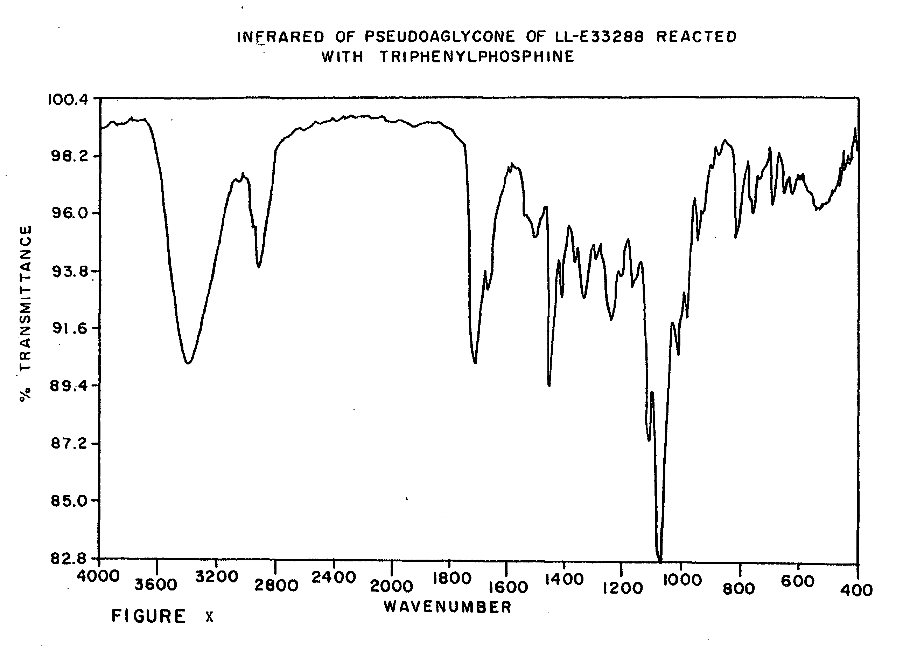

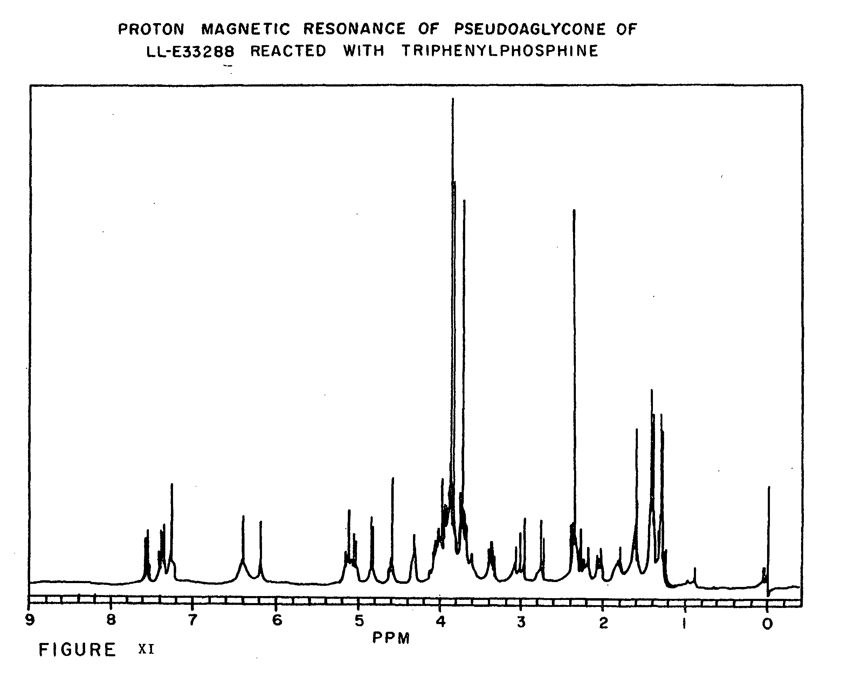

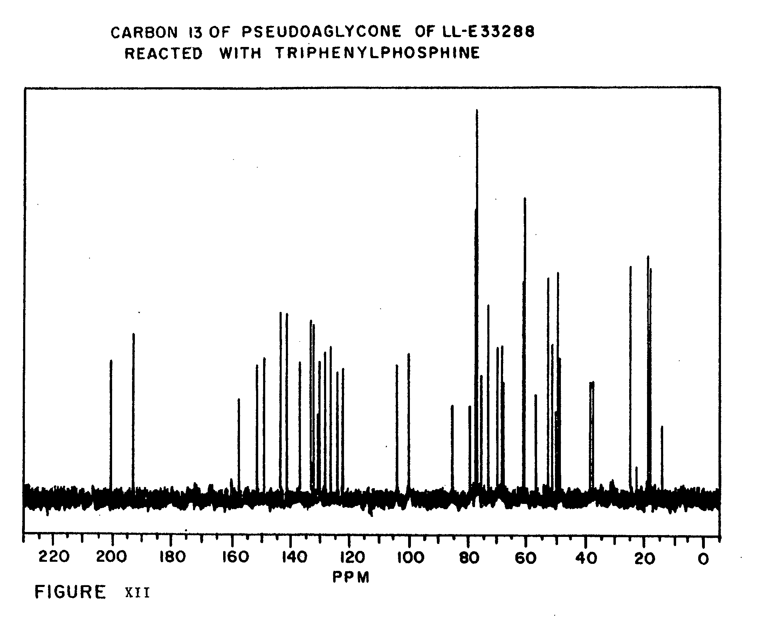

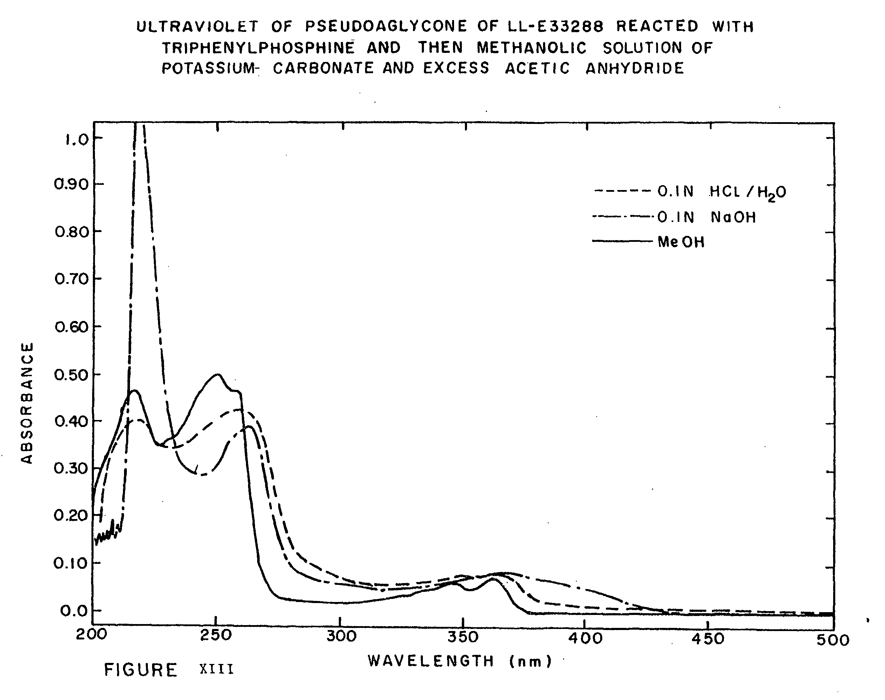

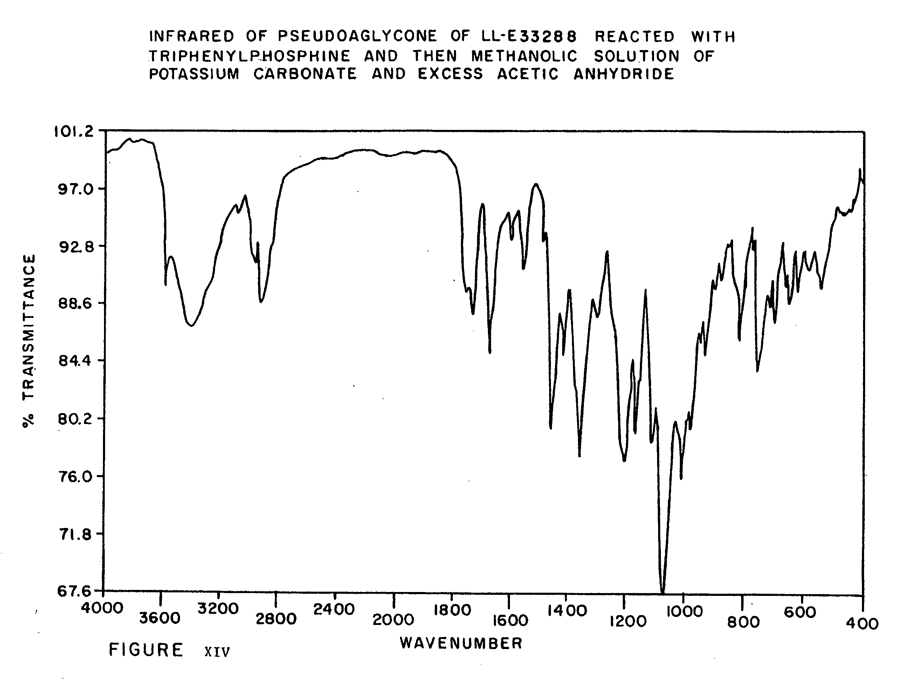

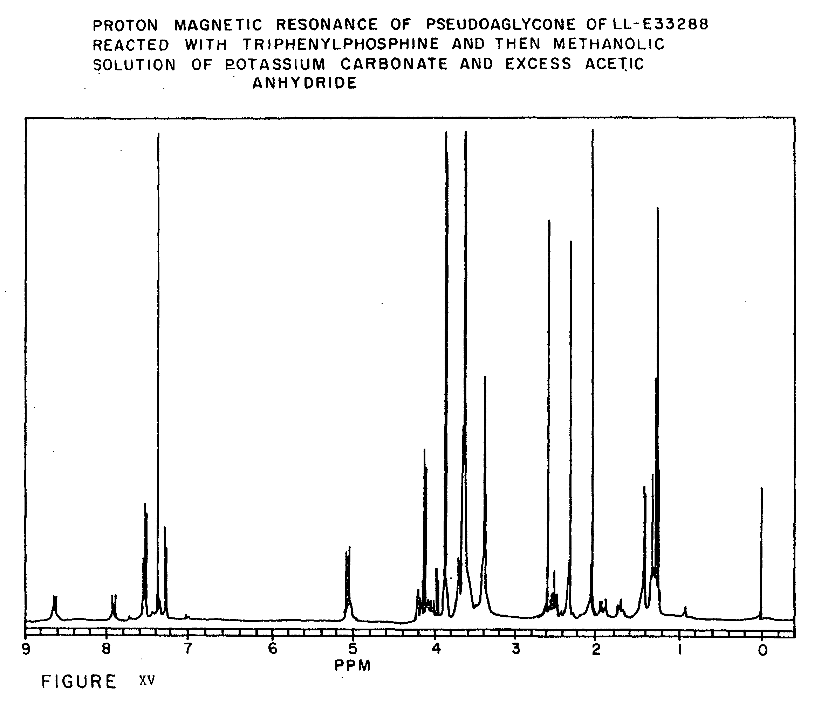

5. Antitumorantibiotikum nach Anspruch 4, das Dihydro-LL-E33288-Pseudoaglykon genannt wird, welches die folgenden physikalisch-chemischen Eigenschaften aufweist:
a) Molekulargewicht 1052 (bestimmt gemäß FAB-MS) ;
b) Ultraviolettabsorptionsspektrum wie in Figur 1 gezeigt (Methanol),
c) Infrarotabsorptionsspektrum wie in Figur II dargestellt (KBr-Scheibe);
d) Protonenmagnetresonanzspektrum wie in Figur III gezeigt (300 MHz, CDCl₃); und
e) Magnetresonanzspektrum für Kohlenstoff 13 wie in Figur IV gezeigt (75 MHz, CDCl₃).

6. Verbindung nach einem der vorhergehenden Ansprüche zur Verwendung beim Behandeln bakterieller Infektionen in Warmblütern.

7. Verbindung nach einem der Ansprüche 1 bis 5 zur Verwendung beim Hemmen des Wachstums von Tumoren in einem Säugetier.

8. Verbindung nach einem der Ansprüche 1 bis 5 zur Verwendung beim Zurückbilden von Leukämie in einem Säugetier.

9. Verwendung einer Verbindung nach einem der Ansprüche 1 bis 5 bei der Herstellung eines Medikaments zur Behandlung bakterieller Infektionen in Warmblütern.

10. Verwendung einer Verbindung nach einem der Ansprüche 1 bis 5 bei der Herstellung eines Medikaments zum Hemmen des Wachstums von Tumoren in einem Säugetier.

## Patentansprüche (Patentansprüche für folgende(n) Vertragsstaat(en): AT , BE , CH , LI , DE , FR, GB, IT, NL, SE)

1. Dihydroderivat des iodierten Pseudoaglykons von LL-E33288 mit der Formel:

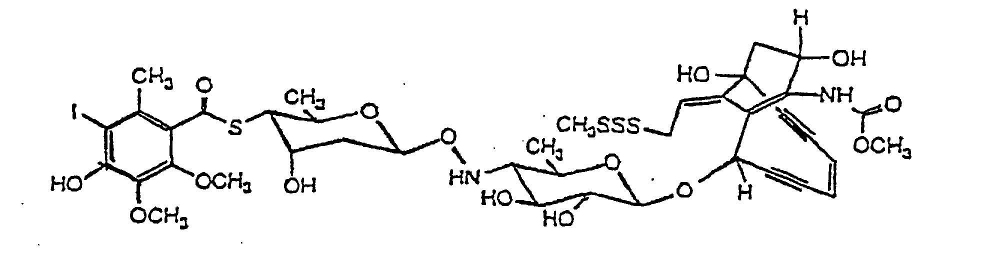

2. Dihydroderivat des bromierten Pseudoaglykons von LL-E33288 mit der Formel:

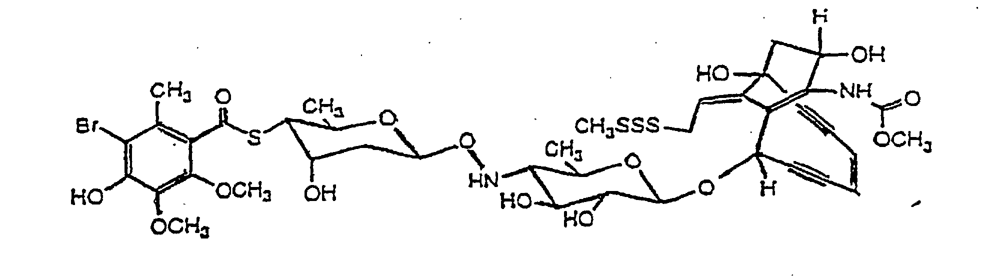

3. Dihydroderivat von LL-E33288α₂-Br mit der Formel: 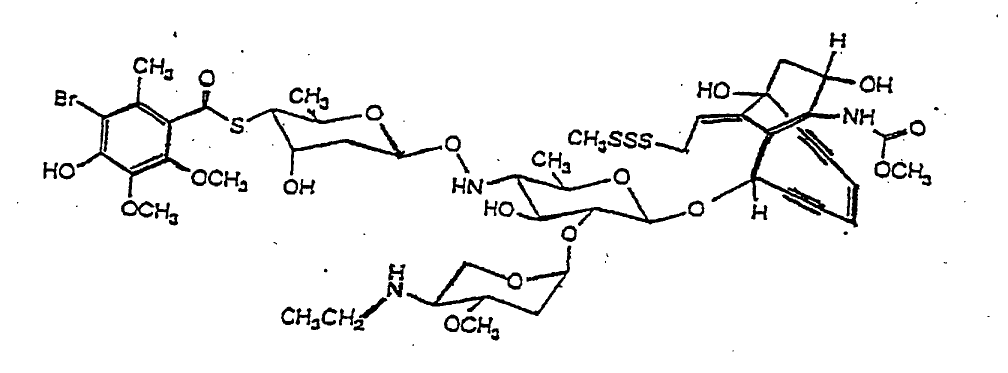
das Dihydroderivat von LL-E33288α₃-Br mit der Formel: 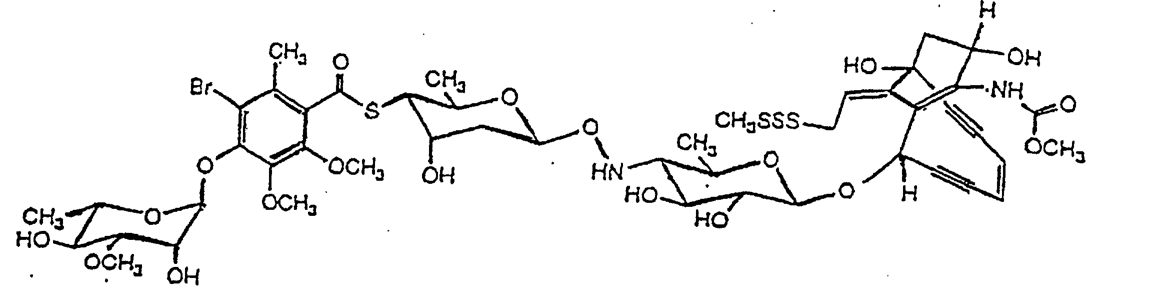
das Dihydroderivat von LL-E33288β₁-Br mit der Formel: 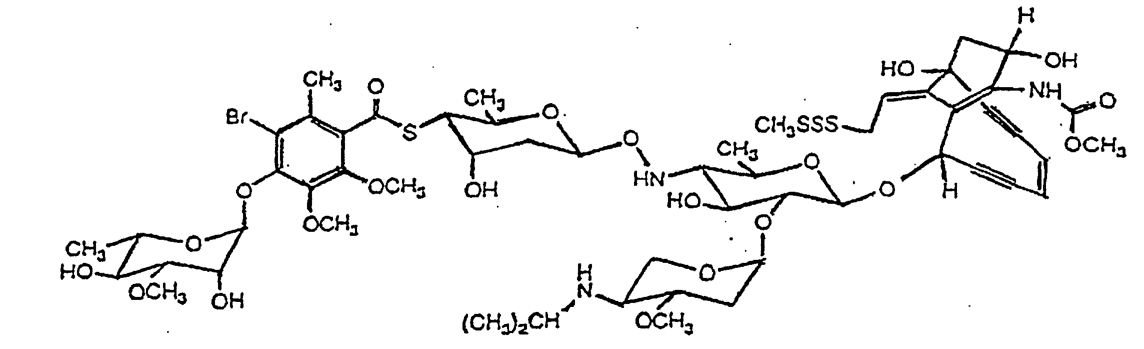
das Dihydroderivat von LL-E33288γ₁-Br mit der Formel: 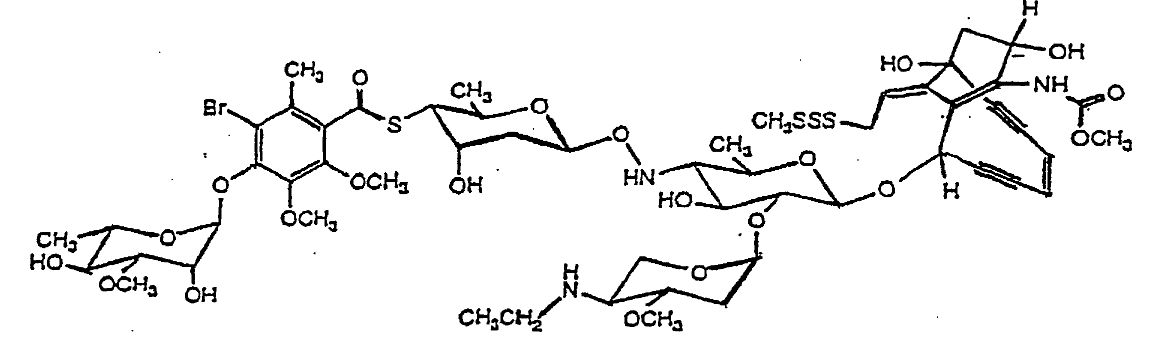
das Dihydroderivat von LL-E33288α₂-I mit der Formel: 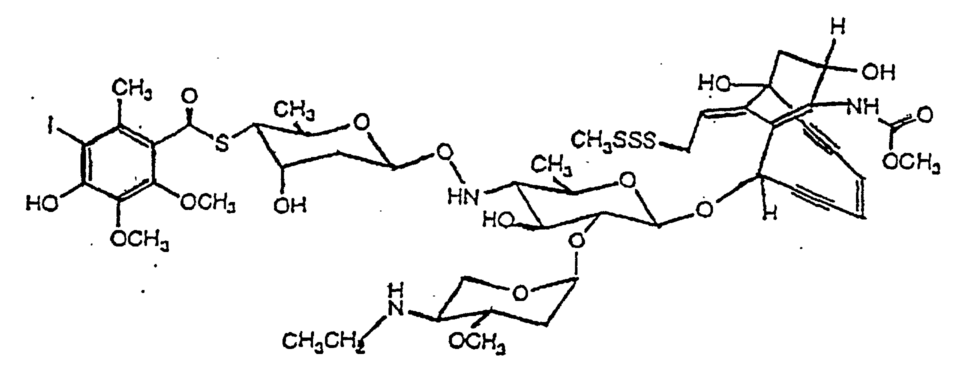
das Dihydroderivat von LL-E33288α₃-I mit der Formel: 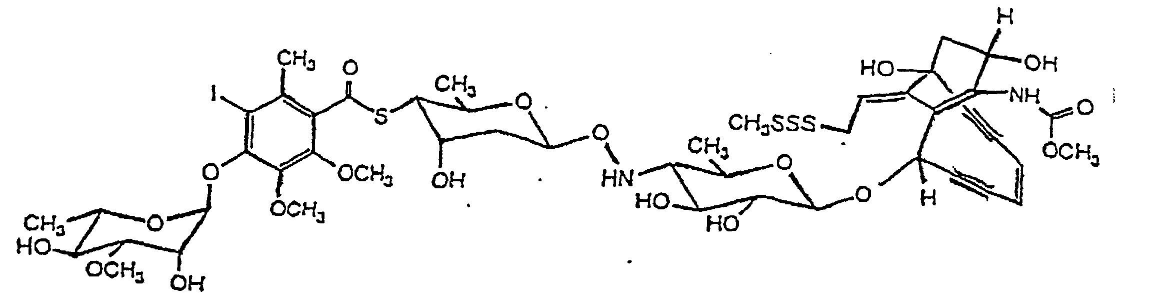
das Dihydroderivat von LL-E33288β₁-I mit der Formel: 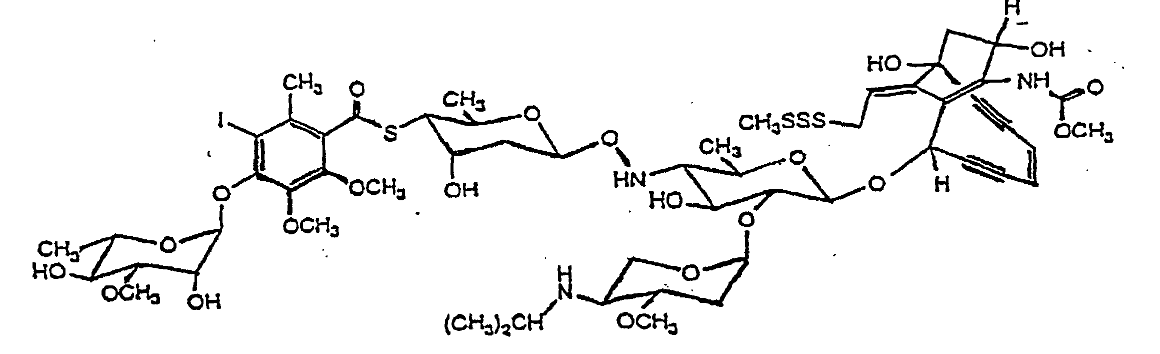
das Dihydroderivat von LL-E33288γ₁-I mit der Formel: 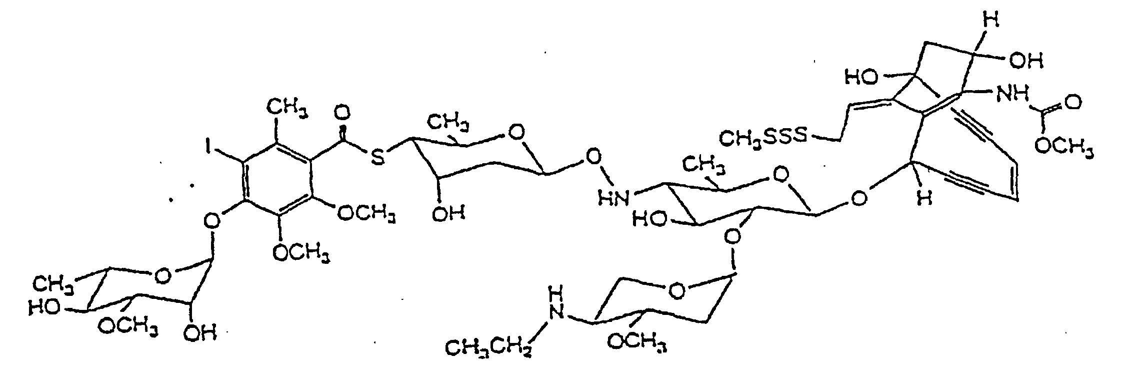
das Dihydroderivat von LL-E33288γ₁-I mit der Formel:

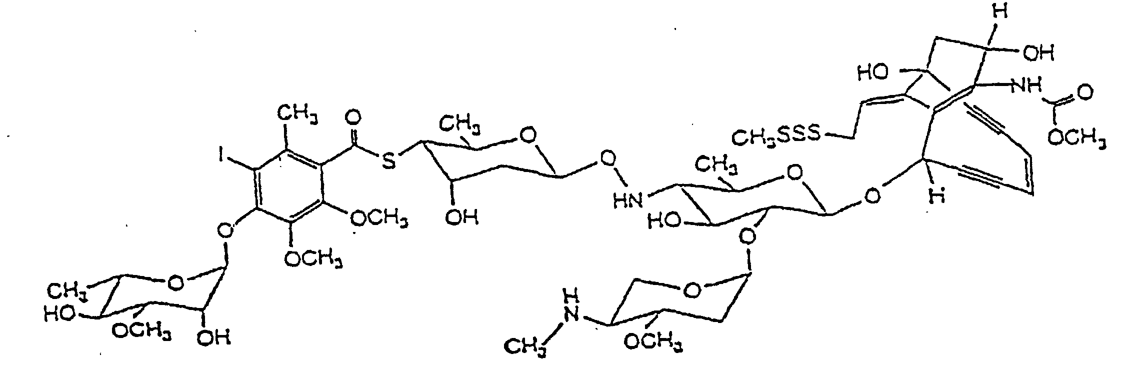

4. LL-E33288-Antitumorantibiotikum, das erhalten werden kann u.a. durch:
aerobes Fermentieren eines flüssigen Mediums, das assimilierbare Quellen von Kohlenstoff, Stickstoff, Iod und anorganischen Salzen enthält, welches Medium mit einer lebensfähigen Kultur des Organismus Micromonosporal echinospora ssp. calichensis von Mutanten NRRL-18149 oder NRRL-15839 oder Mutanten von NRRL-18149 geimpft wurde, bis eine wesentliche antibiotische Aktivität vermittelt ist;
Halten der Fermentationskultur bei einer Temperatur von etwa 24 - 32°C für eine Zeitspanne von ungefähr 90 - 200 Stunden; Ernten der Maische und Rückgewinnen des Antibiotikums; Addieren eines Alkyliodids zu einer Ethanollösung des Antibiotikums; Kühlen der Lösung auf Eisbadtemperatur; Addieren einer Ethanollösung von Natriumborhydrid; Aufschließen des Boratkomplexes durch die Addition von Essigsäure, Ausfällen aus einer Ethylacetatlösung durch die Addition von Hexan und Reinigen durch Chromatographie.

5. Antitumorantibiotikum nach Anspruch 4, das Dihydro-LL-E33288-Pseudoaglykon genannt wird, welches die folgenden physikalisch-chemischen Eigenschaften aufweist:
a) Molekulargewicht 1052 (bestimmt gemäß FAB-MS);
b) Ultraviolettabsorptionsspektrum wie in Figur 1 gezeigt (Methanol),
c) Infrarotabsorptionsspektrum wie in Figur II dargestellt (KBr-Scheibe);
d) Protonenmagnetresonanzspektrum wie in Figur III gezeigt (300 MHz, CDCl₃); und
e) Magnetresonanzspektrum für Kohlenstoff 13 wie in Figur IV gezeigt (75 MHz, CDCl₃).

6. Verbindung nach einem der vorhergehenden Ansprüche zur Verwendung beim Behandeln bakterieller Infektionen in Warmblütern.

7. Verbindung nach einem der Ansprüche 1 bis 5 zur Verwendung beim Hemmen des Wachstums von Tumoren in einem Säugetier.

8. Verbindung nach einem der Ansprüche 1 bis 5 zur Verwendung beim Zurückbilden von Leukämie in einem Säugetier.

9. Verwendung einer Verbindung nach einem der Ansprüche 1 bis 5 bei der Herstellung eines Medikaments zur Behandlung bakterieller Infektionen in Warmblütern.

10. Verwendung einer Verbindung nach einem der Ansprüche 1 bis 5 bei der Herstellung eines Medikaments zum Hemmen des Wachstums von Tumoren in einem Säugetier.

11. Verwendung einer Verbindung nach einem der Ansprüche 1 bis 5 bei der Herstellung eines Medikaments zum Zurückbilden von Leukämie in einem Säugetier.

12. Prozeß zur Herstellung des Dihydroderivats des iodierten Pseudoaglykons von LL-E33288, Dihydroderivats des bromierten Pseudoaglykons von LL-E-33288, Dihydro-LL-E33288α₂-Br, Dihydro-LL-E33288α₃-Br, Dihydro-LL-E33288β₁-Br, Dihydro-LL-E33288γ₁-Br, Dihydro-LL-E33288α₂-I, Dihydro-LL-E33288α₃-I, Diyhdro-LL-E33288β₁-I, Dihydro-LL-E33288γ₁-I oder Dihydro-LL-E33288δ₁-I nach einem der Ansprüche 1 bis 5, welcher ein Addieren eines Alkyliodids zu einer Ethanollösung des Antibiotikums, Kühlen der Lösung auf Eisbadtemperatur, Addieren einer Ethanollösung von Natriumborhydrid, Aufschließen des Boratkomplexes durch die Addition von Essigsäure, Ausfällen aus einer Ethylacetatlösung durch die Addition von Hexan und Reinigen durch Chromatographie umfaßt.

13. Zusammensetzung eines Stoffs in Form einer Einheitsdosierung, mit einer antibakteriell wirksamen Menge einer Verbindung nach einem der Ansprüche 1 bis 5.

## Revendications (Revendications pour l'(les) Etat(s) contractant(s) suivant(s): GR,ES)

1. Dérivé dihydrogéné de la pseudoaglycone iodée de LL-E33288 de formule :

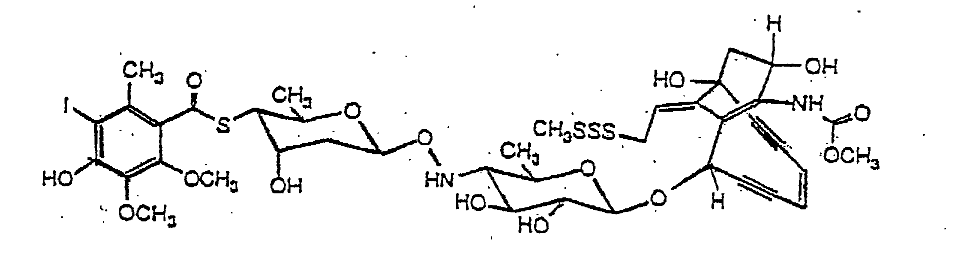

2. Dérivé dihydrogéné de la pseudoaglycone bromée de LL-E33288 de formule :

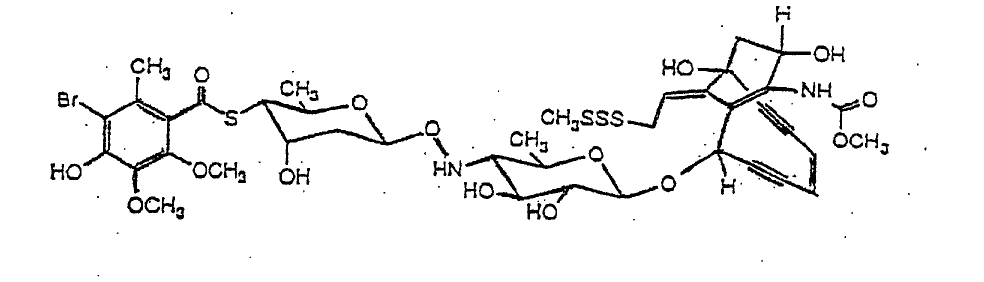

3. Dérivé dihydrogéné de LL-E33288α₂-Br de formule : 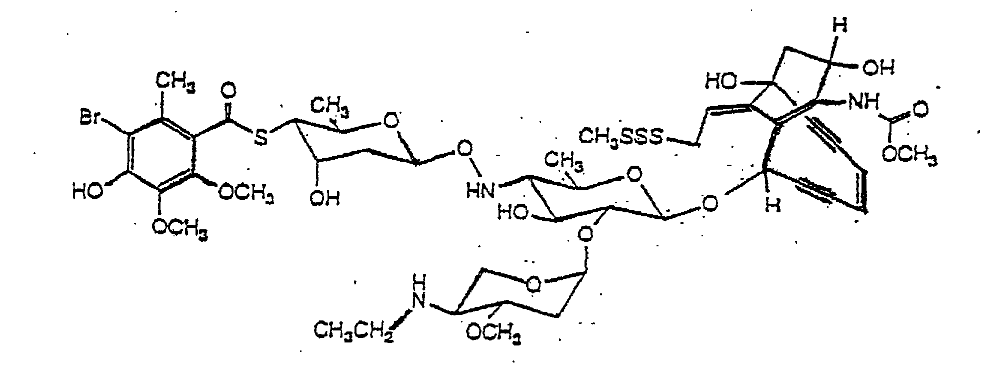
dérivé dihydrogéné de LL-E33288α₃-Br de formule : 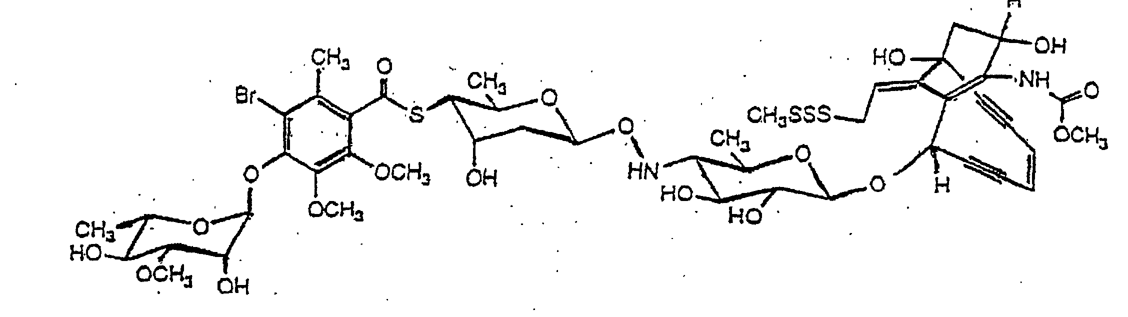
dérivé dihydrogéné de LL-E33288β₁-Br de formule : 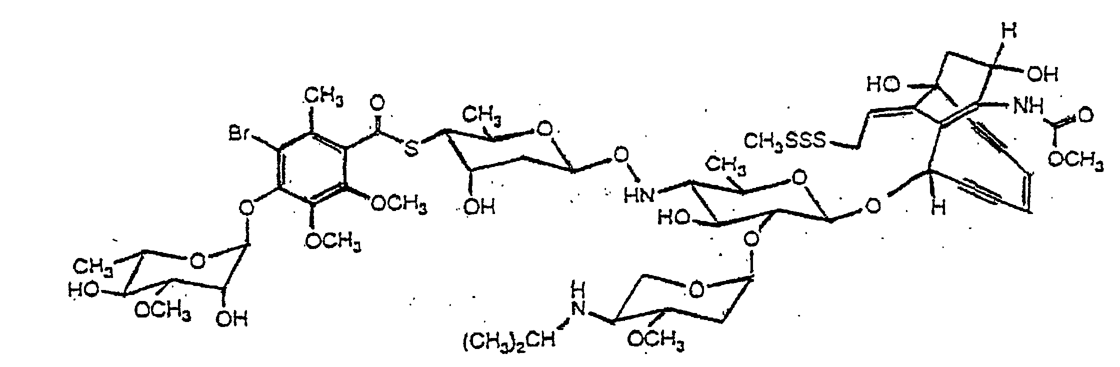
dérivé dihydrogéné de LL-E33288γ₁-Br de formule : 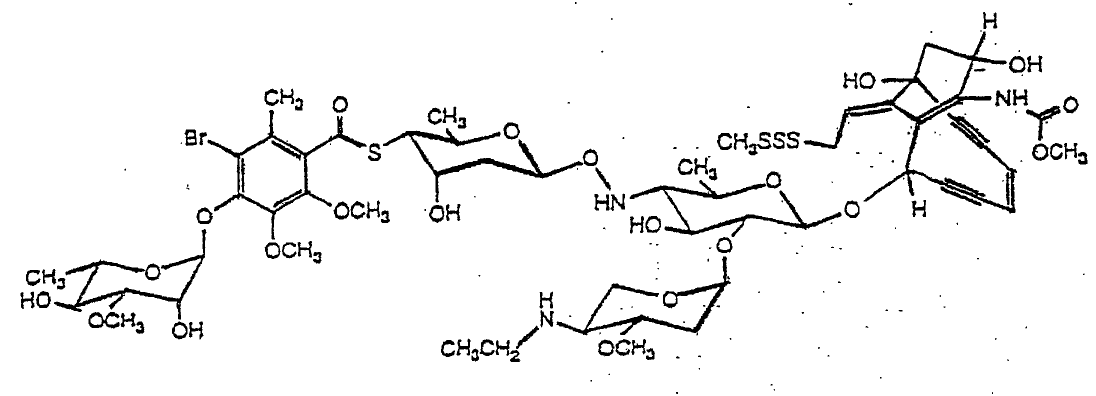
dérivé dihydrogéné de LL-E33288α₂-I de formule : 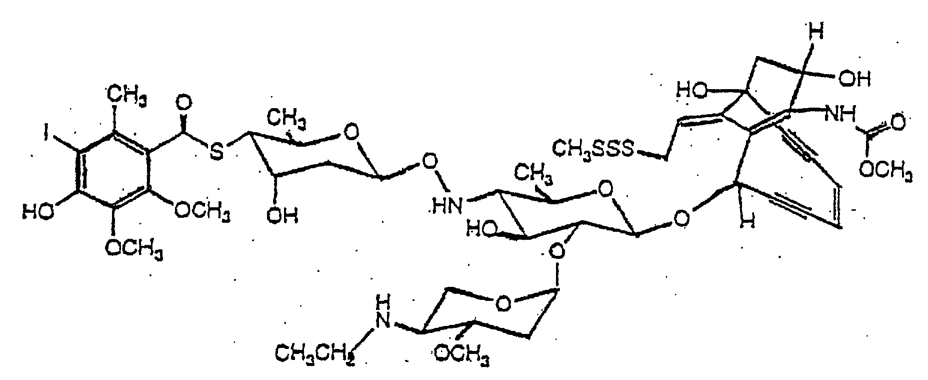
dérivé dihydrogéné de LL-E33288α₃-I de formule : 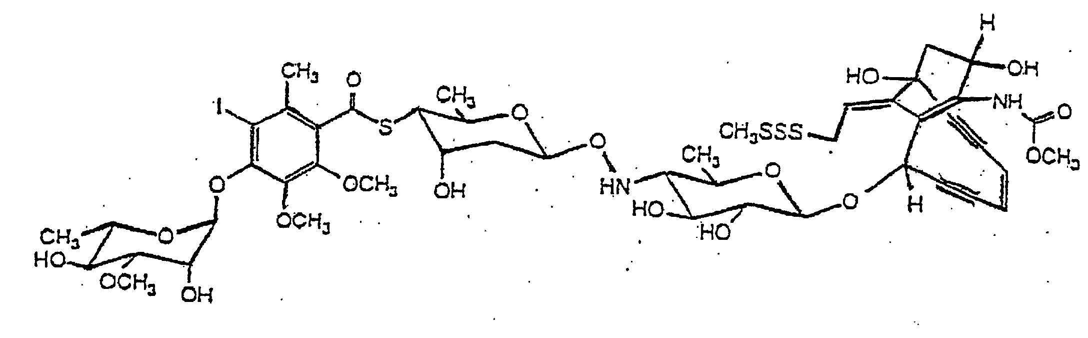
dérivé dihydrogéné de LL-E33288β₁-I de formule : 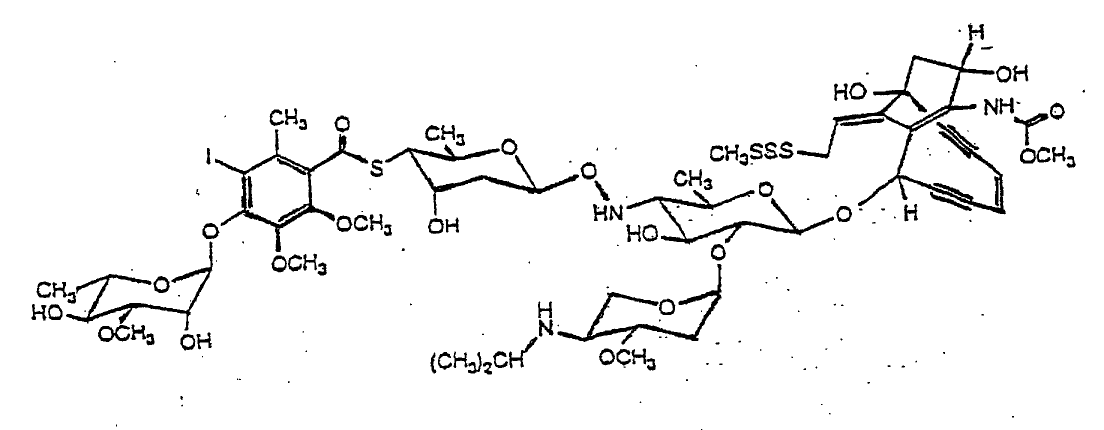
dérivé dihydrogéné de LL-E332888γ₁-I de formule : 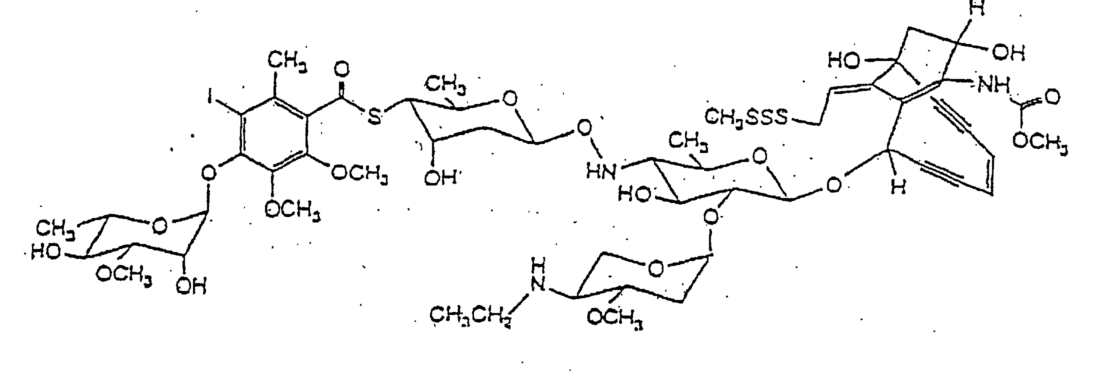
dérivé dihydrogéné de LL-E33288δ₁-I de formule :

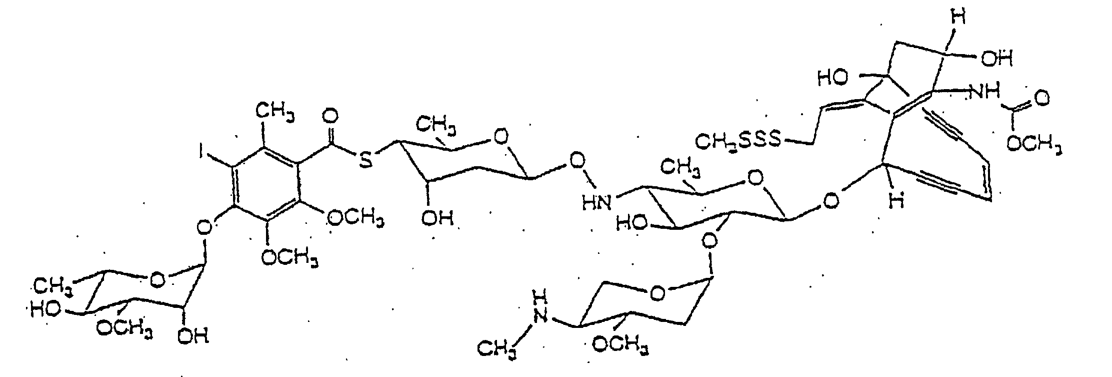

4. Antibiotique antitumoral LL-E33288 pouvant être obtenu, entre autres, par : fermentation aérobie d'un milieu liquide contenant des sources assimilables de carbone, d'azote, d'iode et de sels minéraux, ledit milieu ayant été inoculé avec une culture viable de l'organisme Micromonosporal echinospora ssp. calichensis mutant NRRL-18149 ou NRRL-15839 ou des mutants de NRRL-18149, jusqu'à ce qu'une activité antibiotique substantielle ait été conférée ; maintien de ladite culture en fermentation à une température d'environ 24-32°C pendant une durée d'environ 90-200 heures ; récolte de la trempe et récupération de l'antibiotique ; addition d'un iodure d'alkyle à une solution éthanolique de l'antibiotique ; refroidissement de la solution à la température d'un bain de glace ; addition d'une solution éthanolique de borohydrure de sodium ; décomposition du complexe de borate par addition d'acide acétique, précipitation dans une solution d'acétate d'éthyle par addition d'hexane et purification par chromatographie.

5. Antibiotique antitumoral selon la revendication 4 appelé dihydro-LL-E33288-pseudoaglycone, ayant les caractéristiques physicochimiques suivantes :
a) un poids moléculaire de 1052 (déterminé par spectrométrie de masse à bombardement d'atomes rapides) ;
b) un spectre d'absorption ultraviolette tel que représenté sur la figure I (méthanol) ;
c) un spectre d'absorption infrarouge tel que représenté sur la figure II (pastille de KBr) ;
d) un spectre de résonance magnétique du proton tel que représenté sur la figure III (300 MHz, CDCl₃) ; et
e) spectre de résonance magnétique du carbone-13 tel que représenté sur la figure IV (75 MHz, CDCl₃).

6. Composé selon l'une quelconque des revendications précédentes à utiliser pour traiter des infections bactériennes chez les animaux à sang chaud.

7. Composé selon l'une quelconque des revendications 1 à 5 à utiliser pour inhiber la croissance de tumeurs chez un mammifère.

8. Composé selon l'une quelconque des revendications 1 à 5 à utiliser pour faire régresser la leucémie chez un mammifère.

9. Utilisation d'un composé selon l'une quelconque des revendications 1 à 5 dans la fabrication d'un médicament pour traiter les infections bactériennes chez les animaux à sang chaud.

10. Utilisation d'un composé selon l'une quelconque des revendications 1 à 5 dans la fabrication d'un médicament pour inhiber la croissance de tumeurs chez un mammifère.

## Revendications (Revendications pour l'(les) Etat(s) contractant(s) suivant(s): AT, BE, CH, LI, DE, FR, GB, IT, NL, SE)

1. Dérivé dihydrogéné de la pseudoaglycone iodée de LL-E33288 de formule :

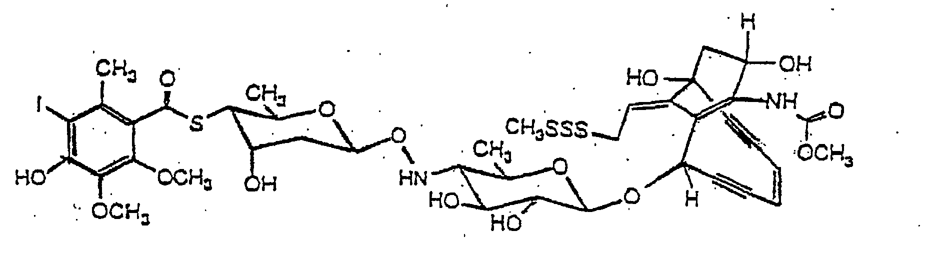

2. Dérivé dihydrogéné de la pseudoaglycone bromée de LL-E33288 de formule :

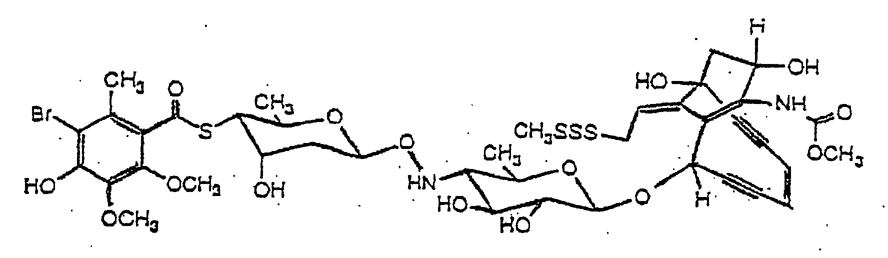

3. Dérivé dihydrogéné de LL-E33288α₂-Br de formule : 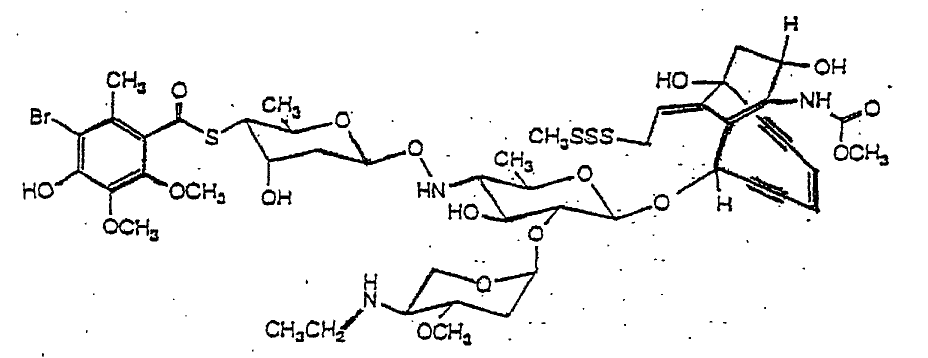
dérivé dihydrogéné de LL-E33288α₃-Br de formule: 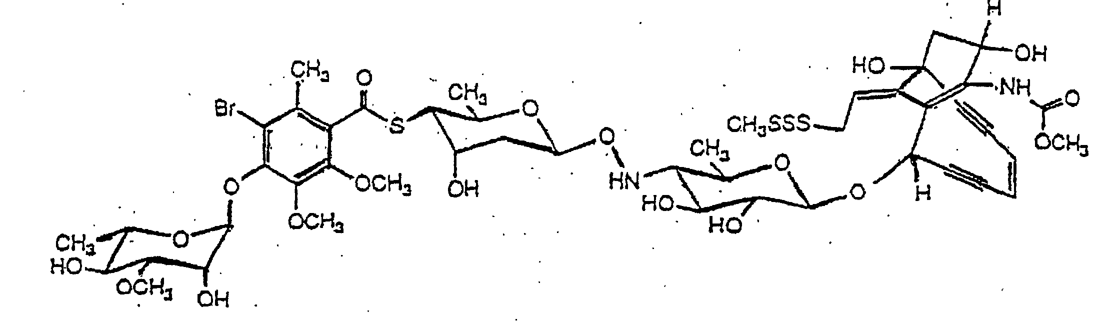
dérivé dihydrogéné de LL-E33288β₁-Br de formule : 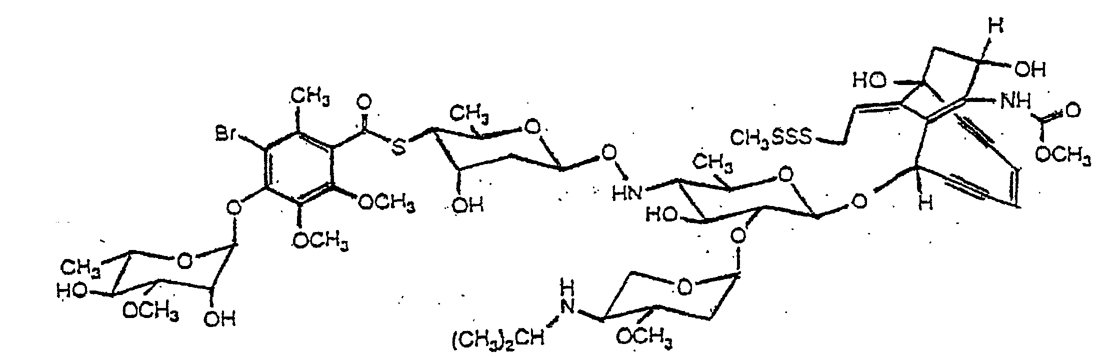
dérivé dihydrogéné de LL-E33288γ₁-Br de formule : 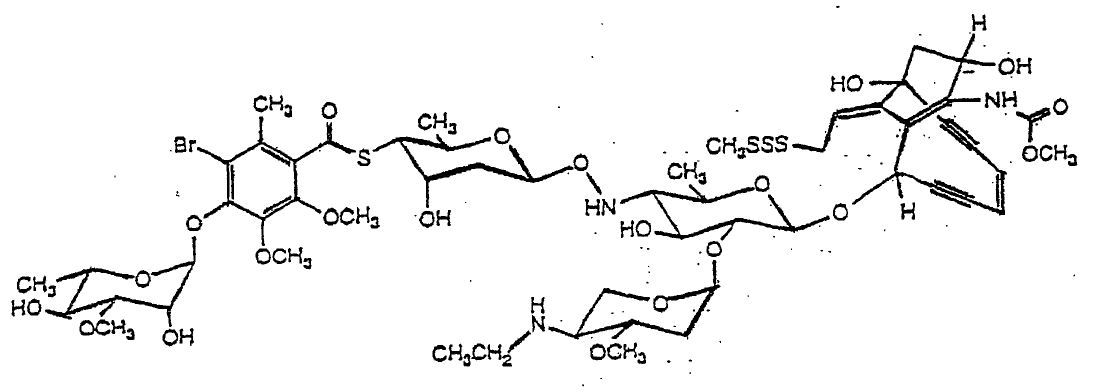
dérivé dihydrogéné de LL-E33288α₂-I de formule : 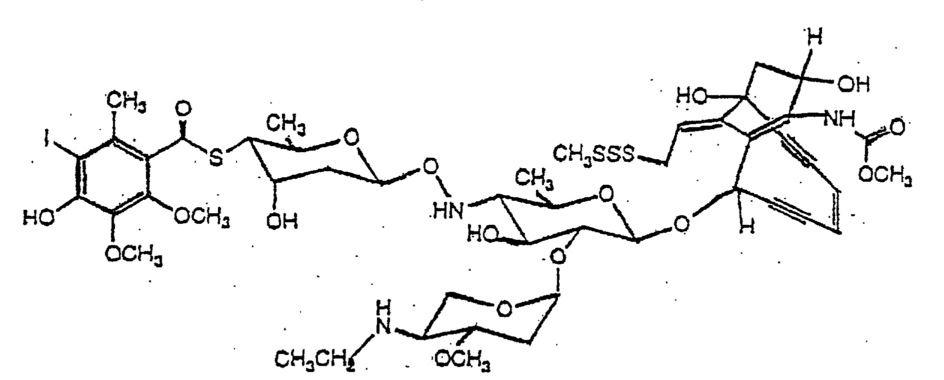
dérivé dihydrogéné de LL-E33288α₃-I de formule : 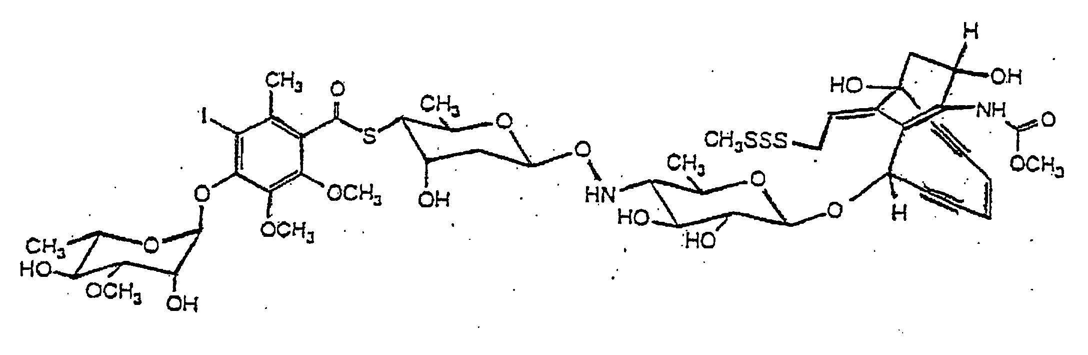
dérivé dihydrogéné de LL-E33288β₁-I de formule : 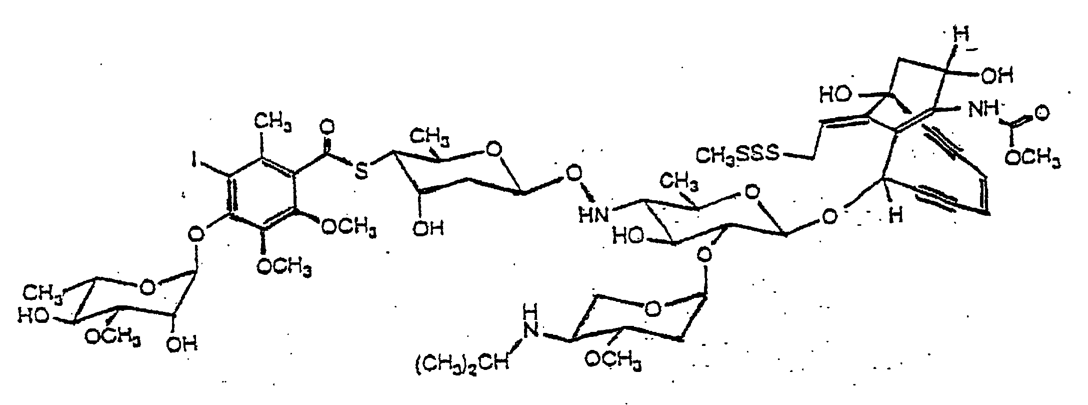
dérivé dihydrogéné de LL-E33288γ₁-I de formule : 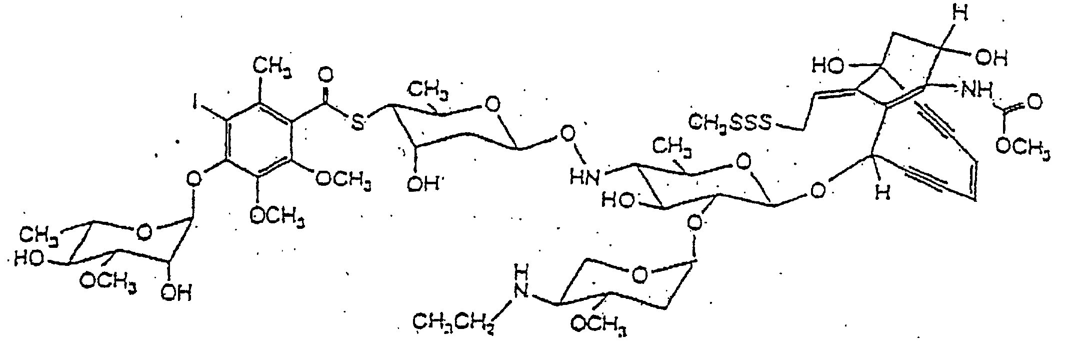
dérivé dihydrogéné de LL-E33288δ₁-I de formule :

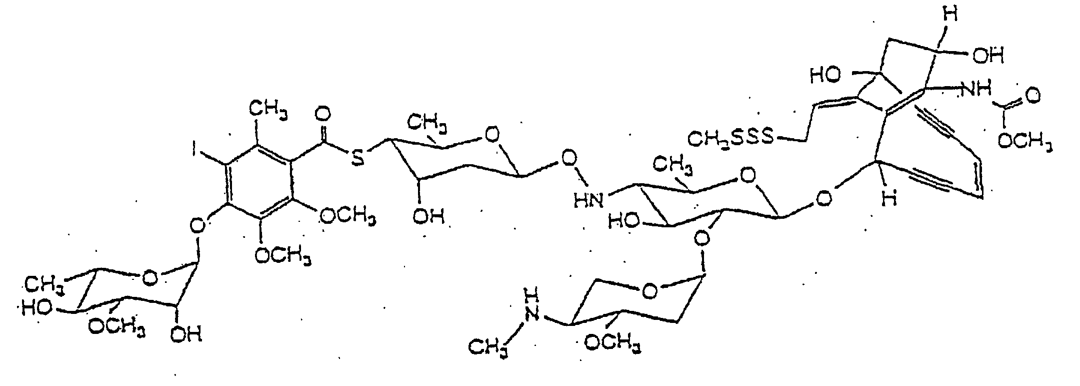

4. Antibiotique antitumoral LL-E33288 pouvant être obtenu, entre autres, par : fermentation aérobie d'un milieu liquide contenant des sources assimilables de carbone, d'azote, d'iode et de sels minéraux, ledit milieu ayant été inoculé avec une culture viable de l'organisme Micromonosporal echinospora ssp. calichensis mutant NRRL-18149 ou NRRL-15839 ou des mutants de NRRL-18149, jusqu'à ce qu'une activité antibiotique substantielle ait été conférée ; maintien de ladite culture en fermentation à une température d'environ 24-32°C pendant une durée d'environ 90-200 heures ; récolte de la trempe et récupération de l'antibiotique ; addition d'un iodure d'alkyle à une solution éthanolique de l'antibiotique ; refroidissement de la solution à la température d'un bain de glace; addition d'une solution éthanolique de borohydrure de sodium; décomposition du complexe de borate par addition d'acide acétique, précipitation dans une solution d'acétate d'éthyle par addition d'hexane et purification par chromatographie.

5. Antibiotique antitumoral selon la revendication 4 appelé dihydro-LL-E33288-pseudoaglycone, ayant les caractéristiques physicochimiques suivantes :
a) un poids moléculaire de 1052 (déterminé par spectrométrie de masse à bombardement d'atomes rapides) ;
b) un spectre d'absorption ultraviolette tel que représenté sur 1a figure I (méthanol) ;
c) un spectre d'absorption infrarouge tel que représenté sur la figure II (pastille de KBr) ;
d) un spectre de résonance magnétique du proton tel que représenté sur la figure III (300 MHz, CDCl₃); et
e) spectre de résonance magnétique du carbone-13 tel que représenté sur la figure IV (75 MHz, CDCl₃).

6. Composé selon l'une quelconque des revendications précédentes à utiliser pour traiter des infections bactériennes chez les animaux à sang chaud.

7. Composé selon l'une quelconque des revendications 1 à 5 à utiliser pour inhiber la croissance de tumeurs chez un mammifère.

8. Composé selon l'une quelconque des revendications 1 à 5 à utiliser pour faire régresser la leucémie chez un mammifère.

9. Utilisation d'un composé selon l'une quelconque des revendications 1 à 5 dans la fabrication d'un médicament pour traiter les infections bactériennes chez les animaux à sang chaud.

10. Utilisation d'un composé selon l'une quelconque des revendications 1 à 5 dans la fabrication d'un médicament pour inhiber la croissance de tumeurs chez un mammifère.

11. Utilisation d'un composé selon l'une quelconque des revendications 1 à 5 dans la fabrication d'un médicament pour faire régresser la leucémie chez un mammifère.

12. Procédé de production du dérivé dihydrogéné de la pseudoaglycone iodée de LL-E33288, du dérivé dihydrogéné de la pseudoaglycone bromée de LL-E33288, du dihydro-LL-E33288α₂-Br, du dihydro-LL-E33288α₃-Br, du dihydro-LL-E33288β₁-Br, du dihydro-LL-E33288γ₁-Br, du dihydro-LL-E33288α₂-I, du dihydro-LL-E33288α₃-I, du dihydro-LL-E33288β₁-I, du dihydro-LL-E33288γ₁-I ou du dihydro-LL-E33288δ₁-I tels que définis dans l'une quelconque des revendications 1 à 5, qui comprend l'addition d'un iodure d'alkyle à une solution éthanolique de l'antibiotique, le refroidissement de la solution à la température d'un bain de glace, l'addition d'une solution éthanolique de borohydrure de sodium, la décomposition du complexe de borate par addition d'acide acétique, la précipitation dans une solution d'acétate d'éthyle par addition d'hexane et la purification par chromatographie.

13. Composition de matière sous forme de dose unitaire comprenant une quantité efficace en tant qu'antibactérien d'un composé selon l'une quelconque des revendications 1 à 5.
